# EUROPEAN PATENT SPECIFICATION

(11) **EP 3 281 941 B1**
(45) Date of publication and mention of the grant of the patent: **24.07.2019**
(21) Application number: 17020330.1
(22) Date of filing: 02.08.2017
(51) Int. Cl.: C07D 249/10, C07F 7/18

(54) **PROCESS FOR PREPARING 2-(5-BROMO-4-(1-CYCLOPROPYLNAPHTHALEN-4-YL)-4H-1,2,4-TRIAZOL-3-YLTHIO)ACETIC ACID**
VERFAHREN ZUR HERSTELLUNG VON 2-(5-BROM-4-(1-CYCLOPROPYLNAPHTHALIN-4-YL)-4H-1,2,4-TRIAZOL-3-YLTHIO)ESSIGSÄURE
PROCÉDÉ DE PRÉPARATION D'ACIDE 2-(5-BROMO-4-(1-CYCLOPROPYLNAPHTALÉN-4-YL)-4H-1,2,4-TRIAZOL-3-YTHIO) ACÉTIQUE

(30) Priority: 11.08.2016 CZ 20160491; 24.08.2016 CZ 20160511; 30.09.2016 CZ 20160611
(43) Date of publication of application: 14.02.2018
(73) Proprietor: Zentiva K.S., 102 37 Praha 10 (CZ)
(72) Inventor: Stach, Jan, 104 00 Praha 10 (CZ); Radl, Stanislav, 250 84 Kvetnice (CZ); Zvatora, Pavel, 198 00 Praha 9 (CZ); Pisa, Ondrej, 378 31 Horni Pena (CZ); Benediktova, Kristyna, 149 00 Praha 4 (CZ); Halama, Ales, 530 09 Pardubice (CZ)
(74) Representative: Jirotkova, Ivana

(56) References cited:
- WO-A1-2014/008295
- CN-A- 104 447 589

## Description

### Field of the Invention

The invention relates to a novel and efficient production methods of 2-(5-bromo-4-(1-cyclopropyl-naphthalen-4-yl)-4*H*-1,2,4-triazol-3-ylthio)acetic acid of formula **1** known under the generic name lesinurad.

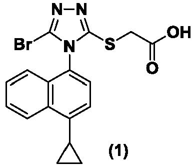

### Background Art

Lesinurad is a selective inhibitor of uric acid reabsorption, which is reducing the urine acid transporter URAT1, thus reducing the level of uric acid in blood. It is used in combination with some of the drugs acting as xanthine oxidase inhibitors in patients where a satisfactory condition is not achieved with xanthine oxidase inhibitors alone.

The medicinal product containing lesinurad is currently approved by the FDA (US Food and Drug Administration) and is commercially available under the tradename Zurampic from the AstraZeneca company. This product contains a dose of 200 mg of free acid in one tablet and is intended for coadministration with xanthin oxidase inhibitors (alopurinol or febuxostat). The approved daily dose is a maximum of 200 mg for one month.

Lesinurad was first described in patent application WO 2006/026356, wherein it was synthetized in five steps from the input naphthylamine of formula **2,** as shown in Diagram 1. Reaction of the naphthylamine of formula **2** with thiophosgene in the first step produces the corresponding isothiocyanate of formula **3,** by its reaction with aminoguanidine and the chromatographic separation in the second step thiol of formula **4** is obtained, but only in 49% yield after 75 hours of reaction time, Alkylation of thiol of formula **4** with a suitable alkyl halogen acetate in the third step results to the ester of formula **5,** which is converted to the lesinurad ester of formula **6** in the next step by a diazotation reaction ongoing in bromoform under very specific conditions (dichloroacetic acid in the presence of benzyltriethylammonium chloride). The final step of the original synthesis of lesinurad of formula **1** is the hydrolysis of the ester of formula **6.**

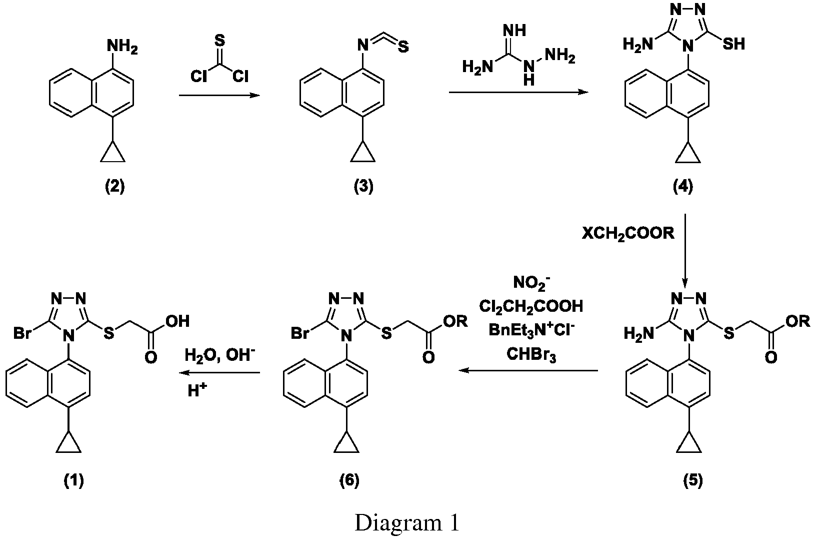

The patent application WO2012092395 describes an alternative conversion of the isothiocyanate of formula **3** to lesinurad of formula **1,** as shown in Diagram 2. The arylthiosemicarbazide of formula **7,** obtained through a reaction of the isothiocyanate of formula **3** with hydrazine, is cyclized with the use of dimethyl carbonate to the thiol of formula **8,** whose reaction with chloroacetic acid provides the acid of formula **9.** Then, the carboxyl group is protected in the form of a suitable ester, the hydroxyl group is substitued by bromine with the use of POBr₃ and the final hydrolysis then provides lesinurad of formula **1.** The procedure is not mentioned in the Examples, but only in diagrams and its yields or other experimental details are not mentioned either.

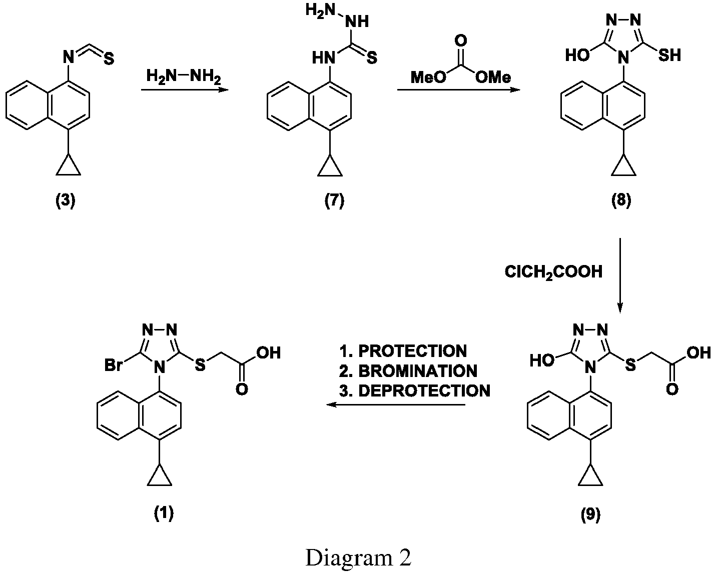

The patent application WO2014008295 describes another option of converting the isothiocyanate of formula **3** to lesinurad of formula **1,** as shown in Diagram 3. The intermediate of formula **10,** obtained through a reaction of the compound of formula **3** with formylhydrazine, is cyclized with potash to the thiol of formula **11.** The ester of formula **12,** obtained by alkylation of thiol of formula **11** by suitable alkyl halogen acetate, is then converted by bromination with the use of *N*-bromosuccinimide (NBS) to lesinurad ester of formula **6,** whose hydrolysis then provides lesinurad **1.**

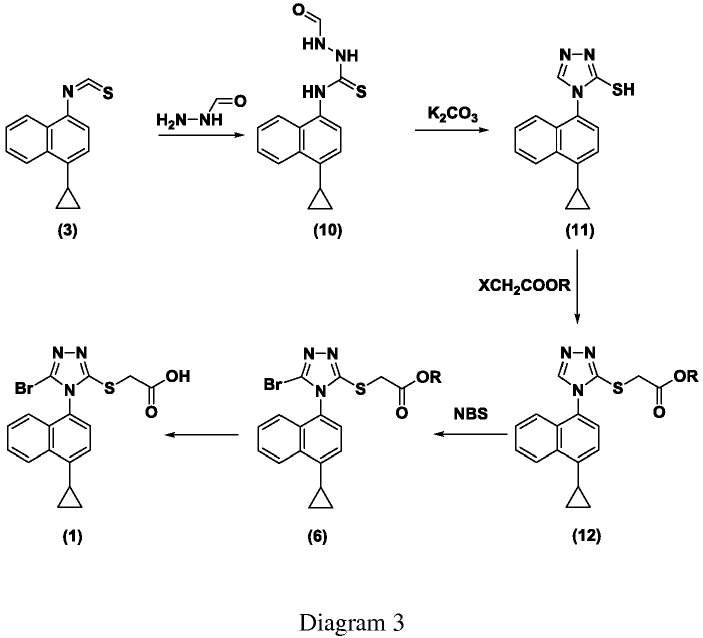

According to applications WO 2014/198241 and WO 2015/054960, some other substrates and alternative methods of bromination may also be used to prepare lesinurad. WO 2014/198241 describes the performance of the brominating reaction on compounds of formula **13,** see Diagram 4. This reaction leads to lesinurad derivatives of general formula **14.** The substituent R¹ in formulas **13** and **14** indicates the groups OR² or NR³R⁴ wherein R² may be C1-C6 alkyl or phenyl, R³ and R⁴ may independently of one another be hydrogen, alkyl or cycloalkyl.

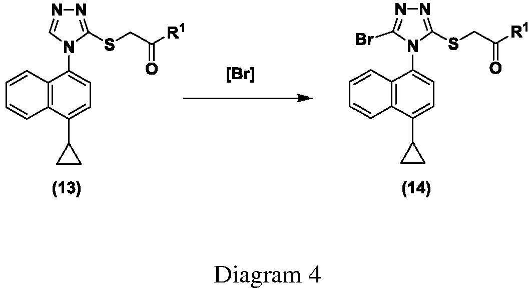

The patent application WO 2015/054960 describes modified bromination of compounds of the general formula **15** using a complex of bromine in pyridine, see Diagram 5. Acetonitrile was used as a suitable solvent. The reaction led to products of the general formula **16** out of which some can be used for the preparation of lesinurad according to the authors, but the experimental details of the reaction of the compound of formula **16** towards lesinurad were not described in more detail. The substituent R in the formulas **15** and **16** e.g. stands for the -COCH₃, -CH₂CN, -CH₂Ph, -CH₂CH₂OH, -CH₂COOMe or -CH₂COOEt groups.

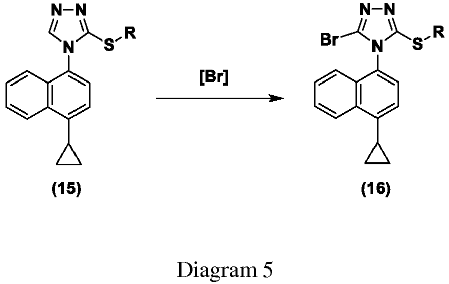

Lesinurad may occur either in the form of free acid or in the form of salts or co-crystals. Solid forms of lesinurad free acid, including polymorphs and solvates, are described for example in the patent applications WO 2012/092395, WO 2015/075561 a WO 2015/095703.

Salts of lesinurad with metals namely with sodium, potassium, magnesium, calcium, zinc and aluminum, are described for example in the patent applications WO 2015/075561, WO 2011/085009, US 2010/0056464, US 2010/0056465, WO 2009/070740, CN 104447589, CN 103755651, WO 2012/050589.

Salts of lesinurad with strong acids, namely with hydrochloric, methanesulfonic, ethanesulfonic, 1,2-ethanedisulfonic and 2-hydroxyethanesulfonic acid are described in the patent application CN 103755651.

Salts of lesinurad with suitable organic cations or neutral molecules able to form co-crystals, such as piperazine, D-glucitol, L-arginine, trichloromethane, 2-methyltetrahydrofuran and 1,5-dihydro-4H-pyrazolo[3,4-*d*]pyrimidin-4-one, are described in the patent applications WO 2015/075561, CN 103755651, WO 2012/050589, WO 2009/070740.

### Disclosure of the Invention

The object of the invention is a method for preparing a compound of formula **I** or its salts, 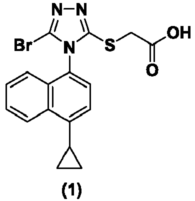 which includes:
**(a)** reaction of acid of formula **17** with the silylating agent in an organic solvent to form an intermediate of formula **18,** wherein R¹, R², R³ are independently of each other C₁-C₂₀ alkyl, C₁-C₂₀ alkenyl, C₃-C₁₀ cycloalkyl, C₃-C₁₀ cycloalkenyl or C₆-C₁₀ aryl; 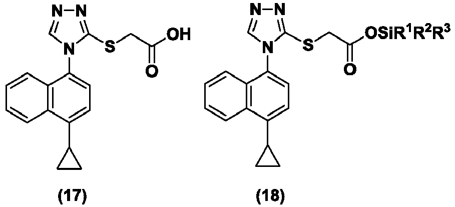
**(b)** bromination of an intermediate of formula **18** with a brominating agent in an organic solvent to form an intermediate of formula **19,** wherein R¹, R², R³ are as defined above; 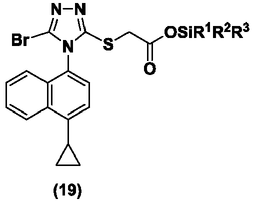
**(c)** deprotection of a trialkylsilyl group from the intermediate of formula **19** to form a compound of formula **1;** and
**(d)** isolation of a compound of formula **1** or its salt.

An intermediate of formula **18** is preferably a trimethylsilylester of form **18a.**

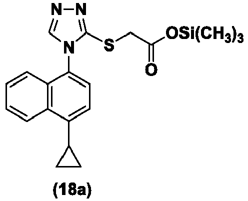

The silylating agents usable to protect the acid of formula **17** include, in particular, those which do not need to add an additional base to successfully complete the protecting reaction. The silylating agent is preferably selected from the group of allyltrimethylsilane, *N,O*-bis(trimethylsilyl)acetamide, bis(trimethylsilyl)carbamate, *N,N*-bis(trimethylsilyl)formamide, *N,N*-bis(trimethylsilyl)methylamine, bis(trimethylsilyl)sulfate, *N*,*O*-bis(trimethylsilyl)trifluoroacetamide, *N,N'*-bis(trimethylsilyl)urea, (ethylthio)trimethylsilane, ethyltrimethylsilyl acetate, hexamethyldisilane, hexamethyldisilazane, hexamethyldisiloxane, hexamethyldisilthiane, (isopropenyloxy)trimethylsilane, methoxy-2-methyl-1-trimethylsiloxypropene, (methylthio)trimethylsilane, methyl-3-trimethylsiloxy-2-butenoate, *N*-methyl-*N*-trimethylsilyl acetamide, methyltrimethylsilyl acetate, *N*-methyl-*N*-trimethylsilyl heptafluorobutyramide, *N*-methyl-N-trimethylsilyl trifluoroacetamide, (phenyl-thio)trimethylsilane, trimethylbromosilane, trimethylchlorosilane, trimethyliodosilane, 4-trimethylsiloxy-3-penten-2-one, *N*-(trimethylsilyl)acetamide, TMS-acetamide, trimethylsilyl acetate, trimethylsilyl azide, trimethylsilyl benzenesulfonate, trimethylsilyl cyanide, *N*-(trimethylsilyl)diethylamine, *N*-(trimethylsilyl)dimethylamine, trimethylsilyl-*N,N*-dimethylcarbamate, 1-(trimethylsilyl)imidazole, trimethylsilyl methanesulfonate, 4-(trimethylsilyl)morpholine, 3-trimethylsilyl-2-oxazolidinone, trimethylsilylperfluoro-1-butanesulfonate, TMS-nonaflate, trimethylsilyl trichloroacetate, trimethylsilyl trifluoroacetate, trimethylsilyl trifluoromethanesulfonate, 2- (trimethylsilyl)ethanol, 2- (trimethyl- silyl)ethoxymethylchloride. In the most preferably embodiment of the invention the silylating agent is *N,O*-bis(trimethylsilyl)acetamide (BSA) or related agents containing two silyls, as BSA is, such as bis(trimethylsilyl)carbamate, *N,N*-bis(trimethylsilyl)formamide, *N,N*-bis(trimethylsilyl)methylamine, bis(trimethylsilyl)sulfate, *N,O*-bis(trimethylsilyl)trifluoroacetamde and *N,N'*-bis(trimethylsilyl)urea.

As an organic solvent for the preparation of intermediates of formulas **18, 18a** and **19** a solvent selected from the group of acetonitrile, dimethylformamide, dimethylsulfoxide, acetone, 2-butanone, 4-methyl-2-pentanone, acetophenone, cyclohexanone, methanol, ethanol, isopropyl alcohol, ethyl acetate, isopropyl acetate, tetrahydrofuran, 2-methyltetrahydrofuran, methylcyclopentyl ether, methyl-t-butyl ether, dichloromethane, cyclohexane, toluene is used. A particularly preferred solvent is tetrahydrofuran.

7 to 16 ml of THF per gram of input substance of formula **17** and 1.1 to 2 equivalents of *N,O*-bis(trimethylsilyl)acetamide as a silylating agent are used to prepare the intermediate of formula **18** or **18a** in the most preferred embodiment.

With regard to its commercial availability and easy handling, *N*-bromosuccinimide (NBS) can be used and is preferred as a suitable brominating agent. However, for the purposes of this reaction, a number of other brominating agents can be used, e.g. pyridinium tribromide (e.g. generated *in situ* during the reaction of bromine with pyridine), benzyltrimethylammonium tribromide, 1,3-dibromo-5,5-dimethylhydantoin (DBDMH), or dibromobarbituric acid.

In the most preferred embodiment, tetrahydrofuran as a solvent and 1.5 to 3 equivalents *N*-bromosuccinimide as a brominating agent are used to prepare an intermediate of formula **19,** preferably at the temperature of 40 to 60°C.

New intermediates of formulas **18, 18a** and **19** are also part of the invention.

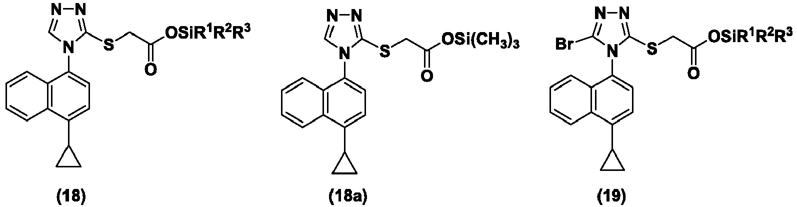

The input acid of formula **17** is preferably prepared by reacting of compound of formula **11** with haloacetic acid, preferably with bromoacetic acid or chloroacetic acid, in the presence of a base in a polar solvent.

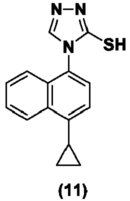

The base is preferably selected from the group of sodium bicarbonate, potassium bicarbonate, sodium carbonate, potassium carbonate, sodium hydroxide, potassium hydroxide, triethylamine, diisopropylethylamine or *N*-methylmorpholine.

The polar solvent is preferably selected from the group of dimethylformamide (DMF), *N,N*-dimethylacetamide, dimethylsulfoxide (DMSO), *N*-methylpyrrolidone (NMP), phosphoric acid tris(*N,N* tetramethylene) triamide, acetonitrile, methanol, ethanol, isopropyl alcohol, water or mixtures thereof.

Deprotection of the trialkylsilyl group from the intermediate of formula **19** is preferably effected by adding water to the solution or extract of the compound of formula **19** in an organic solvent. The compound of formula **1** is then isolated by crystallization from the solution in organic solvents obtained after deprotection of the trialkylsilyl group from the intermediate of formula **19.** Alternatively, isopropylamine or guanidine is added to the solution and the corresponding isopropylamine or suanidine salt is crystallized.

Another object of the invention is therefore also the isopropyl ammonium salt of lesinurad of formula **21,** preferably in crystalline form.

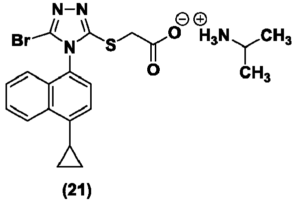

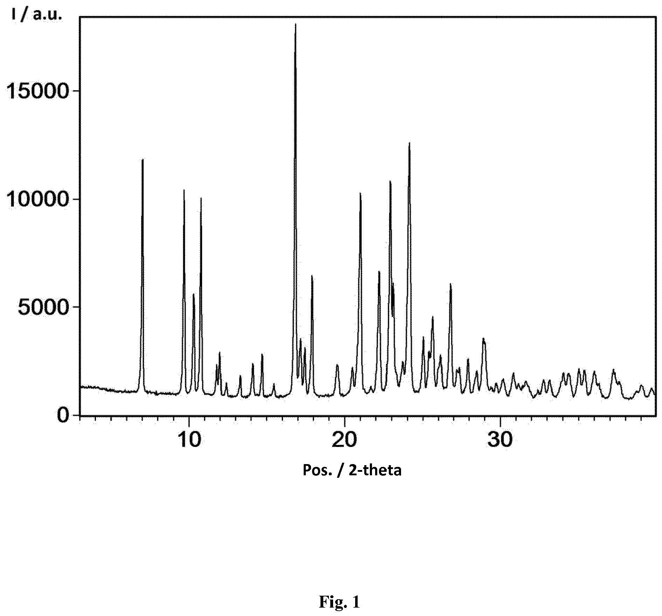

In one embodiment, crystalline lesinurad isopropyl ammonium salt has a characteristic reflection in X-ray powder diffraction, measured using CuKα radiation: 7.0; 16.8; 21.0; 22.9 and 24.1° ± 0.2° 2-theta. Preferably, this salt also has the following diffraction peaks using CuKα radiation: 9.7; 10.8; 17.9; 22.2 and 26.8 ± 0.2° 2-theta. In the most preferred embodiment, the X-ray powder diffraction pattern of the lesinurad isopropyl ammonium salt corresponds with Fig. 1. Reflections with a relative intensity higher than 1% are shown in Table 1.

**Table 1**

| **Position [°2Th.]** | **Interplanar spacing [Å] [Å]=0,1nm** | **Rel. intensity [%]** |
|---|---|---|
| 7,00 | 12,619 | 57,4 |
| 9,66 | 9,146 | 50,8 |
| 10,29 | 8,592 | 26,1 |
| 10,76 | 8,217 | 49,9 |
| 11,97 | 7,388 | 11,3 |
| 13,26 | 6,671 | 5,7 |
| 14,08 | 6,284 | 8,5 |
| 14,68 | 6,029 | 11,7 |
| 16,81 | 5,271 | 100,0 |
| 17,13 | 5,171 | 12,4 |
| 17,41 | 5,089 | 12,4 |
| 17,89 | 4,954 | 32,1 |
| 19,49 | 4,551 | 8,3 |
| 20,47 | 4,336 | 6,6 |
| 20,97 | 4,234 | 50,6 |
| 22,18 | 4,005 | 30,6 |
| 22,90 | 3,881 | 63,8 |
| 23,10 | 3,848 | 25,0 |
| 24,10 | 3,690 | 63,9 |
| 25,02 | 3,557 | 15,9 |
| 25,63 | 3,473 | 20,7 |
| 26,14 | 3,406 | 10,6 |
| 26,75 | 3,329 | 31,7 |
| 27,35 | 3,258 | 7,6 |
| 27,88 | 3,198 | 10,5 |
| 28,43 | 3,137 | 6,2 |
| 28,87 | 3,090 | 14,1 |
| 30,12 | 2,965 | 4,5 |
| 30,77 | 2,903 | 5,1 |
| 31,62 | 2,827 | 5,2 |
| 32,74 | 2,734 | 4,5 |
| 33,98 | 2,636 | 5,7 |
| 34,37 | 2,607 | 5,6 |
| 35,01 | 2,561 | 8,0 |
| 35,36 | 2,536 | 6,7 |
| 35,99 | 2,493 | 6,8 |
| 37,23 | 2,414 | 7,2 |

Lesinurad crystalline isopropyl ammonium salt is further characterized by the melting point of the crystalline form of DSC 156 ± 5°C and a chemical purity determined by HPLC of 99.5% and higher.

Still another object of the invention is lesinurad guanidine salt of formula **22,** preferably in crystalline form.

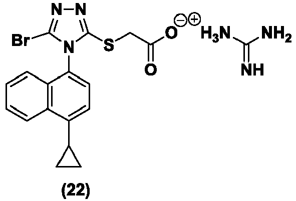

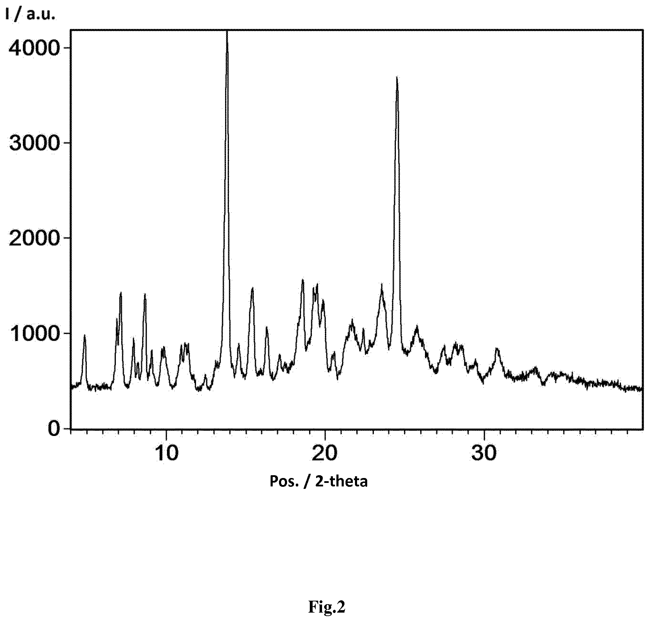

In one embodiment, the lesinurad crystalline guanidinium salt of formula **22a** has characteristic reflections in X-ray powder diffraction pattern, measured using CuKα radiation: 7.1; 13.8; 18.5 and 24.5° ± 0.2° 2-theta. Preferably, this salt also has the following diffraction peaks using CuKα radiation: 8.6; 15.4 and 18.6° ± 0.2° 2-theta. In the most preferred embodiment, the lesinurad guanidinium salt has the X-ray powder diffraction pattern corresponding with the pattern shown in Fig. 2. Reflections with a relative intensity higher than 1% are shown in Table 2.

**Table 2**

| **Position [°2Th.]** | **Interplanar spacing [Å] [Å]=0,1nm** | **Rel. intensity [%]** |
|---|---|---|
| 4,85 | 18,224 | 12,6 |
| 6,88 | 12,836 | 18,0 |
| 7,12 | 12,398 | 27,5 |
| 7,92 | 11,151 | 15,3 |
| 8,62 | 10,245 | 28,7 |
| 9,08 | 9,735 | 10,5 |
| 9,89 | 8,936 | 10,5 |
| 10,91 | 8,102 | 10,8 |
| 11,20 | 7,894 | 9,8 |
| 11,39 | 7,763 | 9,8 |
| 13,79 | 6,417 | 100,0 |
| 14,55 | 6,082 | 10,7 |
| 15,36 | 5,764 | 29,5 |
| 16,31 | 5,430 | 16,9 |
| 17,20 | 5,152 | 5,6 |
| 18,55 | 4,781 | 25,8 |
| 19,43 | 4,566 | 22,5 |
| 19,89 | 4,460 | 17,7 |
| 20,49 | 4,331 | 6,4 |
| 21,53 | 4,124 | 10,5 |
| 22,37 | 3,970 | 9,6 |
| 23,61 | 3,766 | 17,4 |
| 24,48 | 3,633 | 86,7 |
| 25,78 | 3,453 | 11,4 |
| 27,41 | 3,251 | 8,1 |
| 28,11 | 3,171 | 7,1 |
| 28,56 | 3,123 | 7,9 |
| 29,41 | 3,035 | 5,2 |
| 30,81 | 2,900 | 7,5 |

Lesinurad crystalline guanidium salt **22a** is further characterized by the melting point of the crystalline form of DSC 157 ± 5°C and a chemical purity determined by HPLC of 99.5% and higher.

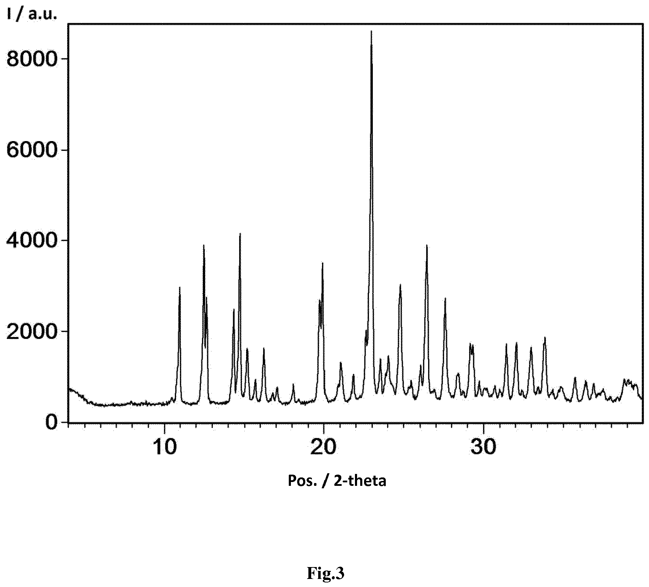

In one embodiment, the lesinurad crystalline guanidine salt **22b** has characteristic reflections in X-ray powder diffraction pattern, measured using CuKα radiation 12.5; 14.7; 19.9; 23.0 and 26.4° ± 0.2° 2-theta. Preferably, this salt also has the following diffraction peaks using CuKα radiation: 10.9; 16.2; 24.7 and 27.6° ± 0.2° 2-theta. In the most preferred embodiment, the lesinurad guanidinium salt has the X-ray powder diffraction pattern corresponding with the pattern shown in Fig. 3. Reflections with a relative intensity higher than 1% are shown in Table 3.

**Table 3**

| **Position [°2Th.]** | **Interplanar spacing [Å] [Å]=0,1nm** | **Rel. intensity [%]** |
|---|---|---|
| 10,92 | 8,094 | 31,9 |
| 12,47 | 7,093 | 49,3 |
| 12,65 | 6,994 | 24,5 |
| 14,33 | 6,177 | 27,9 |
| 14,71 | 6,016 | 47,8 |
| 15,16 | 5,838 | 16,2 |
| 15,67 | 5,652 | 7,0 |
| 16,21 | 5,464 | 16,4 |
| 17,05 | 5,196 | 5,2 |
| 18,07 | 4,905 | 6,5 |
| 19,73 | 4,497 | 32,7 |
| 19,91 | 4,457 | 41,9 |
| 21,06 | 4,216 | 11,2 |
| 21,82 | 4,070 | 8,8 |
| 22,97 | 3,869 | 100,0 |
| 23,51 | 3,780 | 10,7 |
| 24,03 | 3,700 | 13,8 |
| 24,74 | 3,596 | 35,1 |
| 26,02 | 3,422 | 11,1 |
| 26,39 | 3,375 | 44,6 |
| 27,56 | 3,234 | 34,1 |
| 28,32 | 3,149 | 8,9 |
| 29,15 | 3,061 | 20,8 |
| 29,33 | 3,043 | 15,9 |
| 29,72 | 3,004 | 6,9 |
| 30,67 | 2,913 | 5,1 |
| 31,39 | 2,847 | 17,5 |
| 32,01 | 2,794 | 20,1 |
| 32,94 | 2,717 | 17,5 |
| 33,79 | 2,650 | 21,2 |
| 35,68 | 2,514 | 8,8 |
| 36,34 | 2,470 | 6,1 |
| 36,85 | 2,437 | 5,4 |
| 38,77 | 2,321 | 5,3 |
| 39,03 | 2,306 | 6,7 |
| 39,45 | 2,282 | 5,7 |

Lesinurad crystalline guanidium salt **22b** is further characterized by the melting point of the crystalline form of DSC 200 ± 5°C and a chemical purity determined by HPLC 99.5% and higher.

Several analytical methods intended for examination of solid state can be used for distinction of single solid phases, particularly spectral methods, e.g. X-ray powder diffraction pattern (XRPD), or thermoanalytical methods, e.g. differential scanning calorimetry (DSC).

Lesinurad salt of formula **21** or **22** for use as a drug is also part of the invention, particularly for lowering the level of ureic acid in blood, and the pharmaceutical composition comprising lesinurad salt of formula **21** or **22** and at least one pharmaceutically acceptable excipient.

Further disclosed is an alternative method for preparing a compound of formula **1,** 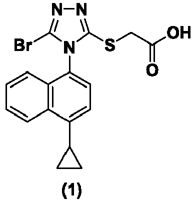 which includes:
**(a)** Reaction of an ester of general formula **5,** wherein R is branched or unbranched C1-C10 alkyl group, preferably methyl, ethyl or tert-butyl group, with a nitrite anion in the presence of a bromine source to form a bromoester of formula **6,** wherein R is as defined above; 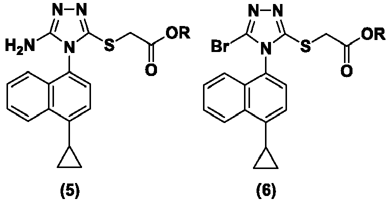
**(b)** alkaline hydrolysis of the bromoester of formula **6.**

The quaternary ammonium bromide of formula **I,** wherein R¹, R², R³ and R⁴ can be independently H, C1-C20 unbranched or branched alkyl groups, unsubstituted or substituted benzyl groups, or unsubstituted or substituted aryl groups, X = Br and where n = 1 to 5 is used as the bromine source.

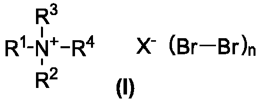

Preferred examples of quaternary ammonium bromides of formula **I** include tetrabutylammonium tribromide, benzyltrimethylammonium tribromide, phenyltrimethylammonium tribromide, pyridinium tribromide, tetrabutylammonium pentabromide and benzyltrimethylammonium pentabromide.

Alternatively, the quarternary ammonium bromide of general formula II, wherein X is Br and R¹, R², R³ and R⁴ can be independently C1-C20 unbranched or branched alkyl groups, unsubstituted or substituted benzyl groups or unsubstituted or substituted aryl groups, can be used, 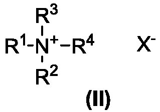 in combination with a brominating agent capable of forming *in situ* adducts with bromine of formula I, such as e.g. bromine or *N*-bromosuccinimide.

The source of nitrite anion to obtain a bromoester of formula **6** is preferably an alkaline nitrite, such as sodium nitrite or potassium nitrite, preferably in the presence of the crown ether, e.g. 15-crown-5, 18-crown-6, or polyethylene glycol, preferably with a molecular weight in the range of 350 to 600, optimally with the molecular weight of 400 (PEG-400).

The reaction is conducted at a temperature of 0°C to the boiling point of the used solvent, preferably at the temperatures form 10°C to 30°C, most preferably at the laboratory temperature. In a preferred embodiment, an acid is added to the reaction of ester of general formula 5, particularly strong organic acid, preferably a halogenated carboxylic acid such as trichloroacetic, dichloroacetic, monochloroacetic, bromoacetic and dibromoacetic acid, or a sulfonic acid such as methanesulfonic, ethanesulfonic, 1,2-ethanedisulfonic, 2-hydroxyethanesulfonic, benzenesulfonic or toluenesulfonic acid. In the most preferred embodiment the reaction is conducted in the presence of methanesulfonic acid.

The following alkaline hydrolysis of an intermediate of formula 6 to the compound of formula **1** is preferably conducted with the use of alkaline hydroxide, such as e.g. LiOH, NaOH, KOH or CsOH, the most preferably with NaOH. Since the reaction conditions of both the stages are compatible, both the stages can be preferably united. The crude lesinurad can be further purified by conversion to a salt with isopropylamine or guanidine, crystallization of such a salt from a suitable solvent and subsequent conversion to a free acid.

Further disclosed is a method of preparing a compound of formula **4,** 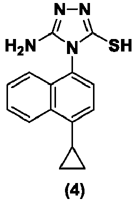 which includes a reaction of 4-cyclopropyl-1-naphthylisothiocyanate of formula **3** with a suitable *S*-alkylthiosemicarbazide of formula **25** in an organic solvent,

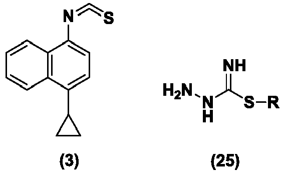

A suitable *S*-alkylthiosemicarbazide of formula **25** is any derivative in which R is C1-C20 branched or unbranched alkyl group or unsubstituted aryl-(C1-C5)alkyl group or aryl-(C1-C5)alkyl group substituted on the aromatic nucleus by one or more branched or unbranched C1-C10 alkyl groups.

In the preferred embodiment the *S*-alkylthiosemicarbazide **25** is in the form of a salt **25a,** 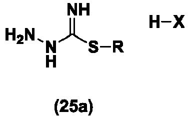 wherein X can be I⁻, Br⁻ or Cl⁻, eventually SO₂OMeO-, where the reaction is conducted in the presence of a base, either organic base such as triethylamine or diisopropylethylamine, metal hydrides, e.g. NaH, metal alkoxides, e.g. sodium or potassium methoxide,sodium or potassium tert-butoxide, sodium or potassium tert-amylate, sodium or potassium hexamethyldisilazide, or potassium 3,7-dimethyl-3-octylate (KDMO), or inorganic bases, such as hydroxides, e.g. lithium, sodium, potassium, cesium hydroxide, carbonates or bicarbonates.

Aromatic carbohydrates are suitable solvents, preferably toluene or C1-C5 unbranched or branched alcohols, aromatic solvents such as carboxylic acids dialkylamides, preferably dimethylformamide (DMF), dimethylacetamide (DMA) or *N*-methylpyrolidone (NMP), carboxylic acids nitriles, preferably acetonitrile, or sulfoxides, preferably dimethylsulfoxide.

The reaction is conducted at a temperature of 0°C to the boiling point of the used solvent, preferably at the temperatures of 10°C to 30°C, most preferably at the laboratory temperature. In a preferred embodiment, the compound of formula **4** is converted without isolation by direct reaction with an alkyl halogenacetate of general formula X-CH₂-COOR, wherein X may be Cl, Br or I, and R is branched od unbranched C1-C10 alkyl group, to a compound of formula **5.**

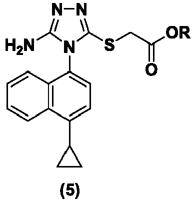

The intermediate of formula **5** is then further processed into lesinurad of formula **1** by any of the known methods or preferably by reaction with quaternary ammonium bromide followed by alkaline hydrolysis, as was described above.

### Detailed description of the Invention

The invention relates to a novel efficient method of preparing 2-(5-bromo-4-(1-cyclopropylnaphthalen-4-yl)-4*H*-1,2,4-triazol-3-ylthio)acetic acid, known under the generic name lesinurad. This method is based on surprising bromination of 2-(4-(1-cyclopropylnaphthalen-4-yl)-4*H*-1,2,4-triazol-3-ylthio)acetic acid of formula **17** protected by the trialkylsilyl group. The whole process is illustrated in Diagram 6, wherein R¹, R² and R³ can independently of one another be alkyl, cycloalkyl or aryl, and X is a suitable leaving group.

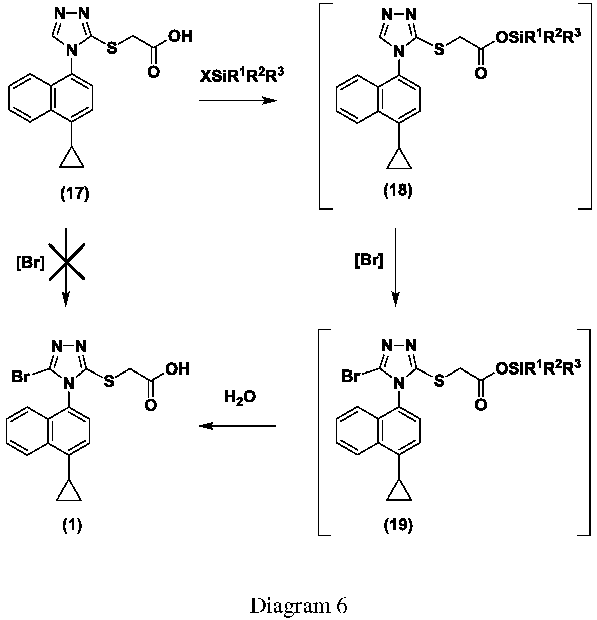

The patent application WO2014008295 (Diagram 3 above) discloses one of the most efficient syntheses of lesinurad of formula 1. A disadvantage of this synthesis is that with regard to instability of the side chain containing sulfur, the bromination only involves the alkyl esters of formula 12 (R is an alkyl) and the products of this step are the alkyl esters of lesinurad of formula 6 exhibiting difficult crystallization behavior. To make isolation of the alkyl esters of formula 6 feasible with a reasonable yield, relatively complicated processing of the reaction mixture is necessary, involving the removal of traces of the unreacted *N*-bromosuccinimide (NBS) and the released succinimide. Besides the problem concerning the isolation of the alkyl esters of lesinurad of formula 6, one more synthetic step is required to obtain the final product. In this case, it is alkaline hydrolysis of the ester with subsequent acidifying of the reaction mixture and isolation of the released lesinurad. Analogous problems may also be associated with alternative brominations of similar compounds 13 (R¹ indicates OR² or NR³R⁴ groups, when R² may be C1-C6 alkyl, or phenyl, R³ and R⁴ may independently of one another be hydrogen, alkyl or cycloalkyl) and 15 (wherein the substitute R indicates e.g. groups selected from the series of -COCH₃, -CH₂CN, -CH₂Ph, -CH₂CH₂OH, -CH₂COOMe or -CH₂COOEt) known from the patent applications WO 2014/198241 and WO 2015/054960 (see Diagrams 4 and 5 above), but isolation of the final lesinurad has not been experimentally described in these cases.

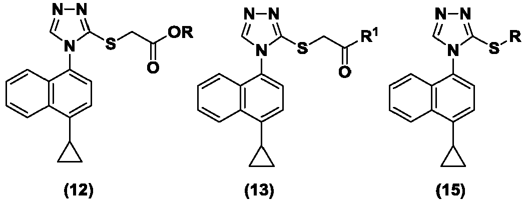

However, analogous bromination reaction of 2-(4-(1-cyclopropylnaphthalen-4-yl)-4*H*-1,2,4-triazol-3ylthio) acetic acid of formula **17,** which is structurally very similar to compounds of formulas **12, 13** and **15,** has not yet been described. Even chemical synthesis of this acid has not been described. The successful use of direct bromination of the acid of formula **17** in the synthesis of lesinurad would offer an advantage in the form of shorter and hence even cheaper synthetic pathway.

However, during attempts at direct bromination of the free acid of formula **17** with suitable brominating agent, e.g. NBS or with bromine-pyridine complex, lesinurad was surprisingly not produced at all, conversely, a complex mixture of especially three majority compounds and a number of further minority impurities was obtained (see Diagram 7). These products are the result of competing reactions running more quickly than the expected bromination reaction. LC-MS analysis of the reaction mixture was used to propose structures of some of these decomposition products of formulas **20a-c.**

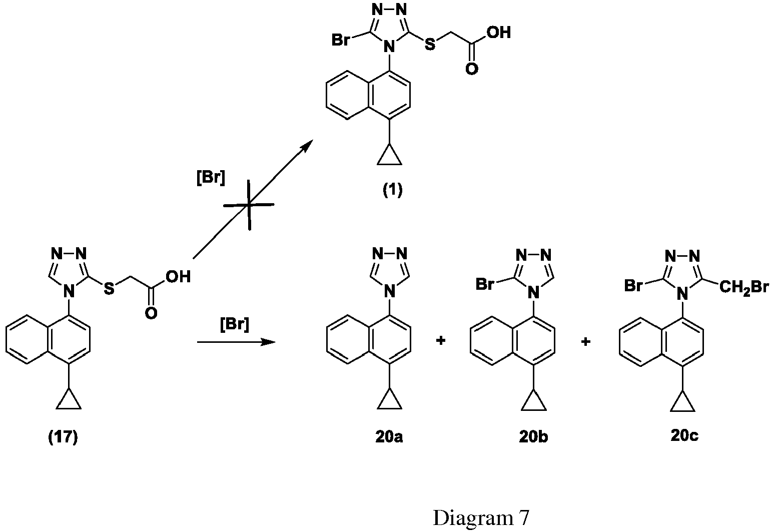

Based on a number of experiments, it has been found that the reaction occurs only when carboxyl functional group is protected by a suitable protecting group. However, the presence of a protecting group also necessitates its later removal and thus a further reaction step. However, the inventors have surprisingly found that an efficient solution to the above-described technical problem is the protection of an acid of formula **17** *in situ* with a trialkylsilyl group. It easily replaces hydrogen in the carboxyl functional group to form an intermediate of formula **18** which is resistant to undesirable reactions and is chemically stable enough as compared to the intermediate of formula **17.** However, the intermediate of formula **19** produced by the subsequent bromination of the compound of formula **18** is labile enough to cleave the protectiong group during the course of the reaction mixture after the bromination is carried out, so that the lesinurad or its crystalline salt is isolated very easily and without the need for a further synthetic step.

The input acid of formula 17 can be prepared from the known intermediate of the synthesis of lesinurad, which is 1-cyclopropyl-4-isothiocyanate naphthalene of formula **3** or the commercially available thiol of formula **11** which is advantageously alkyled with the use of a halogen acetic acid to the acid of formula **17,** see Diagram 8.

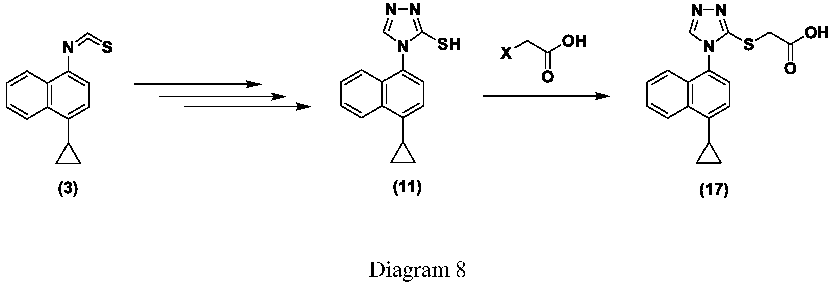

An advantage of the method according to the invention is, among other things, an easy preparation of the trialkylsilyl ester of formula **18** *in situ,* which consisted in addition of the corresponding quantity of a silylating agent to a solution of the acid of formula **17** in an anhydrous organic solvent, preferably in anhydrous tetrahydrofuran (THF), just before the addition of the brominating agent. An especially suitable silylating agent is the commercially available *N,O*-bis(trimethylsilyl)acetamide (BSA) and related compounds, which do not require addition of a supplementary base for successful completion of the protecting reaction.

The bromination reaction itself can be initiated immediately after the addition of a silylating agent to a solution of the acid of formula **17** by adding a suitable brominationg agent to a solution of the trialkylsilyl ester intermediate of formula **18.** The bromination reaction of the acid of formula **17** with the aid of the silylation agents proceeds selectively, but without the addition of silylating agent under the same conditions a complex mixture which does not contain the target product is formed. In addition to the selectivity of the reaction, the raction time required to achieve optimal conversion of the input substance is important too for the bromination of the intermediate of formula **18,** preferably the conversion of 99.9% and higher. A technically advantageous aspect of this bromination reaction is the ability to easily control the reaction rate, the reaction time required for maximum conversion of the input substance, respectively. The reaction rate increases in particular the excess of the brominating agent, the higher concentration of the reactants (i.e. the lower dilution) and the elevated temperature of the reaction mixture. In order to achieve the maximum conversion at an acceptable time, the following conditions were found to be the technically preferred reaction conditions for the system of input substance of formula **17,** solvent (THF), silylating agent (BSA), brominating agent (NBS) and reaction temperature: 1 equivalent of the compound of formula **17,** 7-16 ml of THF per gram of the input substance of formula **17,** 1.1 to 2.0 equivalents of BSA, 1.5 to 3.0 equivalents of NBS and a temperature in the range of 25 to 60°C.

Isolation of the product can be started immediately after the achievement of the optimum conversion within the brominating reaction. This isolation comprises splitting-off of the trialkylsilyl group from the intermediate of formula **19,** which is chemically instable especially in contact with water. This instability is the reason why the intermediate of formula **19** splits off the protecting group during the processing of the reaction mixture after the completed bromination already, so lesinurad or its crystalline salt is directly isolated very easily and without the necessity of another synthetic step.

The final product can be isolated in such a way that the solvent used for the bromination reaction is subjected to vacuum distillation and a solvent suitable for isolation of the product is added to the residue. Subsequently, the obtained solution is washed with water to deprotect the intermediate of formula **19** as well as to wash out impurities that are soluble in water as the residues of the used agents (NBS, BSA), succinimide (released from NBS during the bromination), compounds produced by decomposition of the used agents and residues of solvents soluble in water (THF). The washed solution of lesinurad, free of impurities soluble in water, can be further used to isolate solid lesinurad, or its well-crystallizing salts.

Two novel, well-crystallizing salts of lesinurad have been found by the authors and a process of their crystallization has been developed. These are namely the salt of lesinurad with isopropyl amine of formula **21** and the salt of lesinurad with guanidine of formula **22.** These salts are carrying out better crystallization than lesinurad itself, which results in a higher isolation yield as compared to direct isolation of lesinurad. The second advantage is the purifying effect by the crystallization, increasing the chemical purity of the isolated crystalline salt as the other impurities preferentially remain dissolved in the used solvent. These impurities can be either residues of the reactants and solvents used, but also impurities which are formed from the lack of selectivity of the chemical reactions and the chemical decomposition of products or intermediates.

Further disclosed is a method of preparing a high purity lesinurad of formula **1** from a compound of general formula **5** wherein R is a branched or unbranched C1-C10 alkyl group, see Diagram 9.

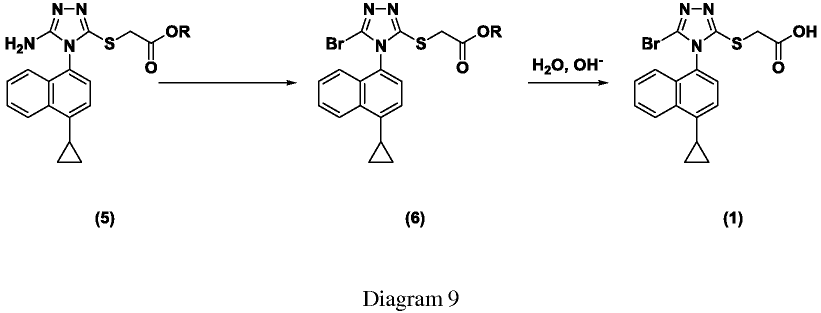

According to the patent application WO 2009/070740, the conversion of the amino derivative of formula **5** to the bromo derivative of formula **6** is carried out with a large excess (20 equivalents) of NaNO₂ in the presence of dichloroacetic acid and benzyltriethylammonium chloride, where the toxic and potentially carcinogenic bromoform is used as the source of bromine. Besides toxicity, it has a disadvantage also at a high boiling point (ranging from 147-151°C). Treatment of the reaction mixture is then accomplished by chromatographic removal of the bromoform, followed by chromatography giving the corresponding ester of formula **6.**

By reproducing the procedure, it has been found that after the hydrolysis of the bromo derivative of formula **6,** the reaction mixture contains, besides lesinurad, a number of impurities. The main impurities are those of formula **17, 23** and **24.**

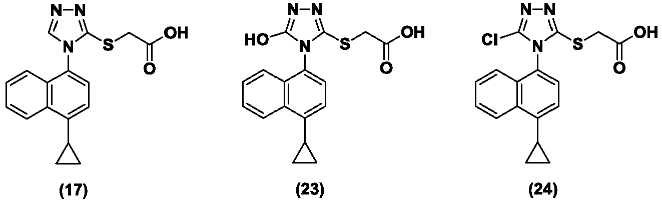

The effort to reduce the quantity of the produced hydroxy impurity of formula **23** by means of diazotization in an anhydrous environment with the use of *tert*-butylnitrite, or *iso*-pentylnitrite led to the generation of a higher quantity of the desbromo impurity of formula **17.**

It was dichloroacetic acid that proved to be the source of the chlorinated impurity of formula **24.** Its replacement with bromoacetic or dibromoacetic acid causes a considerable deceleration of the reaction, but even when benzyltriethylammonium chloride was used, the chloro derivative of formula **24** was not identified. The reaction was accelerated with the use of methanesulfonic acid.

Attempts at replacing bromoform with other sources of bromine usually led to an increase of the content of the desbromo impurity of formula **17** (use of CH₂Br₂, BrCH₂CH₂Br, Br₂CHCHBr₂), or the occurrence of other impurities (use of bromodimethylsulfonium bromide).

It is further disclosed that in case of tetrabutylammonium tribromides and pentabromides such as e.g. tetrabutyl ammonium tribromide, or in case of heptabromides and nonabromides, no other source of bromine needs to be present, while decreasing the amount of impurities.

The best results were achieved with tribromides (tetrabutylammonium tribromide, benzyltrimethylammonium tribromide, phenyltrimethylammonium tribromide, pyridinium tribromide) and with pentabromides (tetrabutylammonium pentabromide, benzyltrimethylammonium pentabromide). Similarly, combinations of tetraalkylammonium bromides with brominating agents capable of generating in situ the said adducts with bromine were tested. The easiest option is the use of the combination of the respective tetraalkylammonium bromide with the corresponding number of bromine equivalents in acetonitrile. From the point of view of reactivity and purity of the final product, the combination of tetrabutylammonium bromide with N-bromosuccinimide, which is known to form a relatively stable complex in suitable solvents, proved to be the most advantageous (J. Org. Chem. 1986, 51, 3548).

A substantial acceleration of the reaction was achieved with the use of the combination of nitrate, such as NaNO₂, with the crown ether, such as 18-crown-6, or polyethylene glycol, polyethylene glycol with the molecular weight preferably in the range of 350 to 600, especially PEG-400.

The hydrolysis of the esters of formula **6** to lesinurad of formula **1** is conducted in an alkaline environment, preferably with the use of alkaline hydroxides such as LiOH, NaOH, KOH or CsOH. Since the reaction conditions of both the stages are compatible, both the stages can be preferably united.

Further disclosed is a method of preparing a compound of formula **4** by the reaction of isothiocyanate of formula **3** with S-alkylthiosemicarbazide **25** or **25a,** as shown in Diagram 10.

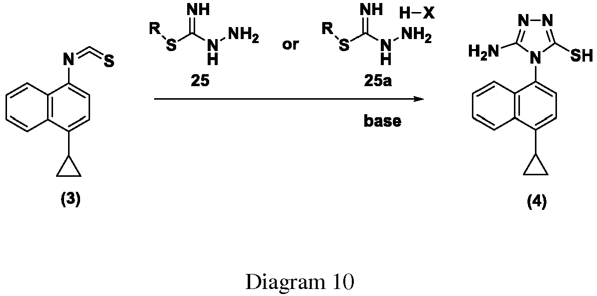

Patent application WO 2009/070740 only shows a low yield of the intermediate of formula **4** obtained by the reaction of an isothiocyanate of formula **3** with 2 equivalents of aminoguanidine using 3 equivalents of diisopropylethylamine as the base in dimethylformamide (DMF) at the temperature of 50°C. After 15 hours of heating, NaOH was added and the mixture was further heated for 60 h. After the treatment, the mixture was separated by chromatography on silica gel and the intermediate of formula **4** was obtained in the yield of 49% (the purity is not stated).

In an attempt to increase the yield of the reaction, the authors have replaced the leaving amino group of aminoguanidine by an alkylsulfanyl group. However, a strongly stinking MeSH was formed during the reaction when a methylsulfanyl group was used. In an attempt to use higher alkyl groups whose reaction provides non-stinking thiols, see, e.g. Node M.: Tetrahedron Lett. 2001, 42, 9207, it was surprisingly found that the reactivity of input substances of formula **25** carrying the bulky alkyl groups R was not significantly lower and was sufficient to perform the reaction at room temperature in all the cases investigated. Among the non-stinking thiols are, e.g. linear thiols of general formula CH₃(CH₂)ₙSH, wherein n is higher than 11, or 4-benzylthiols having one or more tertiary alkyl groups (e.g. *tert*-butyl) or unbranched alkyl groups of general formula CH₃(CH₂)ₙ, wherein n is higher than 5 on the aromatic nucleus.

Thus, the above-described method consists in the reaction of isothiocyanate of formula **3** with *S*-alkylthiosemicarbazide of formula **25** which is preferably liberated *in situ* from the corresponding salt of formula **25a** by treatment with a suitable base. The resulting intermediate of formula **4** is in the next step alkylated with the suitable alkyl halogene acetate to the ester of formula **5.** Since the reaction conditions of both the stages are compatible, both the stages can be preferably united. The ester of formula **5** can subsequently be used as the input compound for the synthesis of lesinurad as described above.

The input S-alkylthiosemicarbazide of formula **25** can be easily obtained by the known method (see, e.g. W. Schulze et al., Pharmazie 1985, 40, 540; H. Emilsson, J. Heterocyclic Chem. 1989, 26, 1077; S. Markovic et al., Tetrahedron 2010, 66, 6205) in the form of corresponding salts of formula **25a** by the reaction of thiosemicarbazide with the corresponding R-X alkyl halide. Low cost commercially available substances such as methyl iodide, dodecyl bromide, octadecyl bromide, benzyl bromide, 4-*tert*-butylbenzyl bromide and 4-octylbenzyl chloride can be used as the input alkyl halides.

### Brief description of the Drawings

**Fig. 1** XRPD pattern of the crystalline salt of lesinurad with isopropylamine of formula **21,** prepared according to Example 11. The pattern illustrates dependence of diffused radiation intensity on the 2-theta diffraction angle.
**Fig. 2** XRPD pattern of the crystalline salt of lesinurad with guanidine of formula **22a,** prepared according to Example 14. The pattern illustrates dependence of diffused radiation intensity on the 2-theta diffraction angle.
**Fig. 3** XRPD pattern of the crystalline salt of lesinurad with guanidine of formula **22b,** prepared according to Example 15. The pattern illustrates dependence of diffused radiation intensity on the 2-theta diffraction angle.

### Examples

The invention is further clarified in the embodiment examples below. These examples only have an illustrative character and are not intended to restrict the scope of the invention in any respect.

### EXAMPLE 1

### N-(4-cyclopropylnaphthalen-1-yl)thiosemicarbazide of formula 7

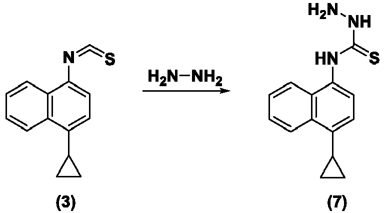

1-cyclopropyl-4-isothiocyanatonaphthalene of formula **3** (17.5 g) was dissolved in tetrahydrofuran (5 ml). After addition of a solution of hydrazine monohydrate (8.8 g) in ethanol (60 ml), the mixture was stirred for 20 minutes at 25°C and the separated product was aspirated and dried. The amount of 18 g (87.5%) of the product was obtained, HPLC purity 98.9%.

### EXAMPLE 2

### 4-(4-cyclopropylnaphthalen-1-yl)-4H-1,2,4-triazole-3-thiol of formula 11

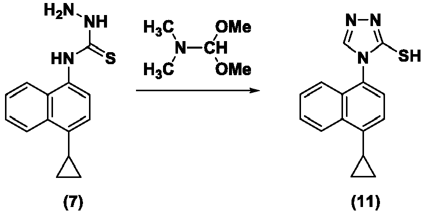

To a solution of *N*-(4-cyclopropylnaphthalen-1-yl)thiosemicarbazide of formula **7** (21.5 g) in tetrahydrofuran dimethyl formamide-dimethyl acetal (20 g) was first added and after 3 h of stirring at 25°C, ethyl acetate (80 ml) and salt brine (50 ml). The separated product was filtered off and dried. The amount of 19.7 g of the compound (90%) was obtained, HPLC purity 99.5%. ¹H NMR (DMSO-D6), δ (ppm): 1.70 (m, 2H), 1.10 (m, 2H), 2.45 (m, 1H), 7.25 (d, 1H), 7.31 (d, 2H), 7.49 (t, 1H), 7.61 (t, 1H), 8.05 (s, 1H), 8.45 (d, 1H). ¹³C NMR (DMSO-D6), δ (ppm): 6.7, 12.9, 122.6, 124.2, 125.5, 125.9, 126.0, 130.1, 132.5, 133.4, 139.0, 142.0, 167.5.

### EXAMPLE 3

### 2-(4-(1-cyclopropylnaphthalen-4-yl)-4H-1,2,4-triazol-3-ylthio)acetic acid of formula 17

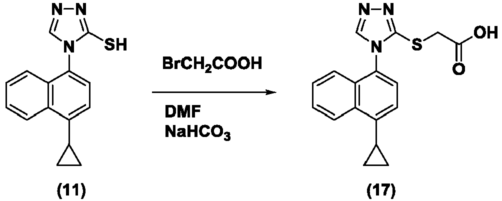

20 ml od DMF and 5 g of NaHCO₃ were added to 5.4 g of 4-(1-cyclopropylnaphthalen-4-yl)-4*H*-1,2,4-triazole-3-thiol of formula **11.** The suspension was stirred at 25°C, 5 g of bromoacetic acid was added and the mixture was stirred for 3 hours. Then, 20 ml of water, 10 ml of ethyl acetate, 30 ml of 2M hydrochloric acid were added and the mixture was stirred for 30 minutes. After that, the separated product was filtered, the cake was washed with water (2x20 ml), ethyl acetate (2x10 ml), ether (2x10 ml) and the product was dried in a vacuum drier. The amount of 6.1 g of the compound (93%) was obtained with the purity of 99.5% (HPLC). ¹H NMR (DMSO-D6), δ (ppm): 0.88 (m, 2H), 1.14 (m, 2H), 2.55 (m, 1H), 4.01 (s, 2H), 7.21 (d, 1H), 7.41 (d, 1H), 7.55 (d, 1H), 7.64 (t, 1H), 7.75 (t, 1H), 8.57 (d, 1H), 8.89 (s, 1H), 12.94 (bs, 1H). ¹³C NMR (DMSO-D6), δ (ppm): 7.0, 7.3, 13.0, 122.1, 122.6, 125.0, 125.5, 127.2, 127.4, 127.8, 128.8, 133.4, 142.3, 146.5, 150.5, 169.3.

### EXAMPLE 4

### 2-(4-(1-cyclopropylnaphthalen-4-yl)-4H-1,2,4-triazol-3-ylthio)acetic acid of formula 17

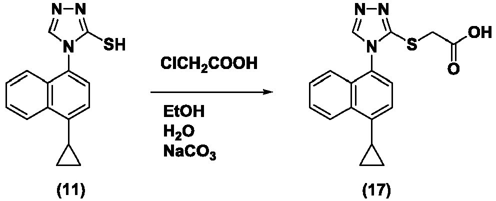

A solution of sodium carbonate (1.1 g) in water (25 ml) was added to a stirred suspension of 4-(1-cyclopropylnaphthalen-4-yl)-4*H*-1,2,4-triazole-3-thiol of formula **11** (2.7 g, 10 mmol) in ethanol (25 ml) and the mixture was stirred for 30 minutes at the laboratory temperature. Then, a solution obtained by dissolution of chloroacetic acid (1.13 g, 12.5 mmol) in water (10 ml) neutralized with sodium carbonate (1.35 g, 12.7 mmol) was added by dripping in 15 minutes. The obtained reaction mixture was stirred for 4 hours at the laboratory temperature and then at the temperature of 50°C. After complete conversion of the input compound of formula **11** according to HPLC, the reaction mixture was cooled and acidified with 10% HCl. The insoluble fraction was aspirated, washed with water and dried in a vacuum drier. The amount of 3.1 g of the compound (95%) was obtained at the HPLC purity of 97.3%, which was used for the subsequent reaction without further purification.

### EXAMPLE 5

### 2-(4-(1-cyclopropylnaphthalen-4-yl)-4H-1,2,4-triazol-3-ylthio)acetic acid of formula 17

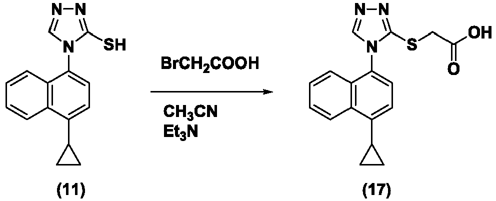

30 ml of acetonitrile, 5 ml of triethylamine and a solution of bromoacetic acid (5 g) in acetonitrile (20 ml) were added to 5.4 g of 4-(1-cyclopropylnaphthalen-4-yl)-4*H*-1,2,4-triazole-3-thiol of formula **11.** The mixture was stirred for 4 hours at 50°C. Then, 40 ml of water, 10 ml of 1M hydrochloric acid were added and the mixture was stirred for 30 minutes. After that, the separated product was filtered, the cake was washed with water (2x20 ml), diethyl ether (2x10 ml) and the product was dried in a vacuum drier. The amount of 5.7 g of the compound (87%) was obtained with the purity of 98.1% (HPLC).

### EXAMPLE 6

### 2-(4-(1-cyclopropylnaphthalen-4-yl)-4H-1,2,4-triazol-3-ylthio)acetic acid of formula 17

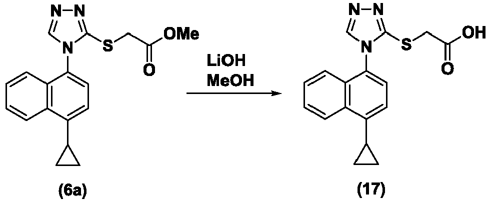

20 ml of methanol, 0.4 g of lithium hydroxide were added to 4 g of the compound of formula **6a** and the mixture was stirred for 60 minutes at 45°C. Then, the mixture was cooled down to 25°C and acidified by addition of 2M of hydrochloric acid. The solvent was evaporated by vacuum, dichloromethane (25 ml) was added to the residue and the suspension was stirred for 10 minutes at the boiling point. Then, filtration was carried out and the cake was washed with 10 ml of dichloromethane. The filtration cake was mixed with 50 ml of water, the suspension was stirred at 65°C and it was acidified to pH 1 to 2 by addition of 2M hydrochloric acid. Then, filtration was carried out, the cake was washed with water (2x25 ml), ethyl acetate (2x15 ml), diethyl ether (2x25 ml) and dried by vacuum. The amount of 3.4 g of white powder (89%) was obtained with the purity of 99.2% (HPLC).

### EXAMPLE 7

### 2-(5-bromo-4-(1-cyclopropylnaphthalen-4-yl)-4H-1,2,4-triazol-3-ylthio)acetic acid of formula 1

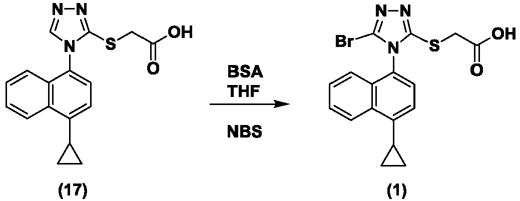

2-(4-(1-cyclopropylnaphthalen-4-yl)-4*H*-1,2,4-triazol-3-ylthio)acetic acid of formula **17** (1 g) was stirred up in tetrahydrofuran (10 ml), *N,O*-Bis(trimethylsilyl)acetamide (1.9 g) and *N*-bromosuccinimide (0.8 g) were added. The solution was stirred at 25°C for 24 h, after addition of 0.3 g of potassium hydroxide in water (10 ml), the solution was shaken with toluene (5 ml), the aqueous phase was acidified with acetic acid (0.4 g) and extracted with ethyl acetate (10 ml), after evaporation and crystallization from ethanol, the amount of 1.14 g (92%) of lesinurad of formula **1** was obtained.

### EXAMPLE 8

### 2-(5-bromo-4-(1-cyclopropylnaphthalen-4-yl)-4H-1,2,4-triazol-3-ylthio)acetic acid of formula 1

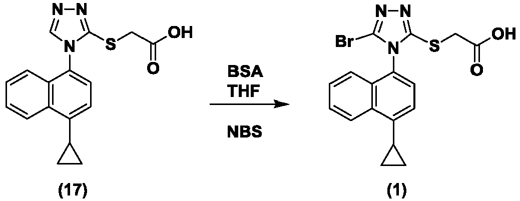

Tetrahydrofuran (THF) and *N,O*-bis(trimethylsilyl)acetamide (BSA) were added to 2-(4-(1-cyclopropylnaphthalen-4-yl)-4*H*-1,2,4-triazol-3-ylthio)acetic acid of formula **17.** The mixture was stirred at 25°C for 5 to 10 minutes and monitored by means of HPLC. Then, *N*-bromosuccinimide (NBS) was added, the obtained solution was stirred, heated up to the selected temperature and the progress of the reaction (conversion of the input compound) was monitored with HPLC.

### Optimization experiments:

(a) Reaction conditions: 15.5 ml of THF per g of the input compound, 2 equivalents of BSA, 2 equivalents of NBS, temperature 25°C, conversion of the input compound 30 minutes after addition of NBS was approx. 22%, after 20 hours 85%.
(b) Reaction conditions: 7.7 ml of THF per g of the input compound, 1.1 equivalents of BSA, 3 equivalents of NBS, temperature 40°C, conversion of the input compound 30 minutes after addition of NBS was approx. 100%.
(c) Reaction conditions: 7.7 ml of THF per g of the input compound, 1.25 equivalents of BSA, 1.5 equivalents of NBS, temperature 40°C, conversion of the input compound 30 minutes after addition of NBS was approx. 65%, after 90 minutes approx. 100%.
(d) Reaction conditions: 7.7 ml of THF per g of the input compound, 1.25 equivalents of BSA, 1.5 equivalents of NBS, temperature 50°C, conversion of the input compound 30 minutes after addition of NBS was approx. 91%, after 90 minutes approx. 100%.

### EXAMPLE 9

### Bromination of the acid of formula 17 without the use of BSA, comparative experiment

Tetrahydrofuran (7.7 ml of THF per g of the input compound) was added to 2-(4-(1-cyclopropylnaphthalen-4-yl)-4*H*-1,2,4-triazol-3-ylthio)acetic acid of formula **17.** The mixture was stirred at 25°C for 5 to 10 minutes and monitored by means of HPLC. Then, *N*-bromosuccinimide (3 equivalents) was added, the obtained solution was stirred, heated up to the selected temperature (40°C) and the course of the reaction (conversion of the input compound) was monitored with HPLC. The conversion of the input compound was approx. 99% 30 minutes after the addition of NBS, but a complex mixture was formed, not lesinurad of formula **1.**

### EXAMPLE 10

### Screening of amines for obtention lesinurad ammonium salt

Lesinurad of formula **1** (0.2 g) was dissolved in 10 ml of acetone, the amine (1.1 equivalents) was added and the mixture was stirred at 25°C. The separated product was filtered off, washed with acetone and dried by vacuum. Crystallization results for the tested amines: *n*-propylamine (the product does not crystallize), *iso*-propylamine (the product crystallizes, yield 93.4%), di-propylamine (the product does not crystallize), *t*-butylamine (the product does not crystallize), benzylamine (the product does not crystallize), cyklohexylamine (the product does not crystallize), lysine (suspension - did not pass through a solution), nicotinamide (the product does not crystallize), glycine (suspension - did not pass through a solution), cyclopentylamine (an amorphous solid compound), meglumine (the product does not crystallize), tromethamine (the product does not crystallize), guanidine (the crystalline product).

The result show that crystallization ability is not a general feature of all lesinurad ammonium salts. From the nitrogen bases investigated, crytsallization was surprisingly achieved only with lesinurad salts with isopropylamine and guanidine, while the other amines tested did not crystallize.

The purity of lesinurad crystalline ammonium salts was compared with the purity of the free acid crystals. The results reported in Table 4 show that the selected salts of lesinurad crystallize to form a product cleaner than the free acid alone.

**Table 4**

| **Lesinurad isolated form** | **Purity according to HPLC** |
|---|---|
| free acid **(1)** | 97% |
| isopropylammonium salt **(21)** | 99,7% |
| guanidine salt **(22a)** | 99,6% |
| guanidine salt **(22b)** | 99,7% |

### EXAMPLE 11

### Isopropylammonium salt of 2-(5-bromo-4-(1-cyclopropylnaphthalen-4-yl)-4H-1,2,4-triazol-3-ylthio)-acetic acid of formula 21

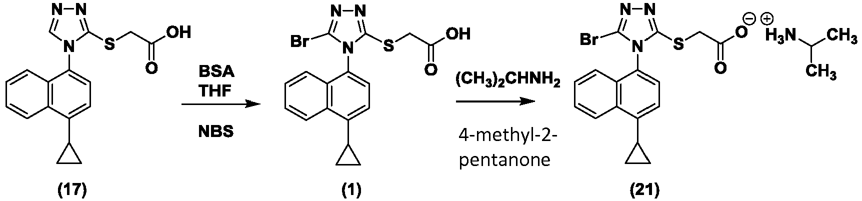

Tetrahydrofuran (25 ml) and *N,O*-bis(trimethylsilyl)acetamide (3.1 ml, 1.25 equivalents) were added to 2-(4-(1-cyclopropylnaphthalen-4-yl)-4*H*-1,2,4-triazol-3-ylthio)acetic acid of formula **17** (3.25 g). The mixture was stirred at 25°C for 5 to 10 minutes and monitored by means of HPLC. Then, *N*-bromosuccinimide (2.67 g, 1.5 equivalents) was added, the obtained solution was stirred, heated up to the selected temperature (50°C) and the course of the reaction (conversion of the input compound) was monitored with HPLC. The conversion of the input compound of formula **17** was approx. 100%, 90 minutes after the addition of NBS. 4-methyl-2-pentanone (100 ml) was added to the reaction mixture, THF was evaporated by vacuum, the obtained solution was washed with water (50 ml), a solution of 10% sodium thiosulfate (25 ml), again with water (50 ml) and dried over sodium sulfate. Then, the drying agent was filtered, isopropylamine (2 ml) was added to the filtrate and this mixture was stirred at 25°C. The separated product was filtered off, washed with 4-methyl-2-pentanone (20 ml) and dried by vacuum. The amount of 3.96 g of the salt (86%) was obtained with the purity of 99.7% (HPLC). ¹H NMR (DMSO-D6), δ (ppm): 0.88 (m, 2H), 1.11 (d, 6H), 1.14 (m, 2H), 2.54 (m, 1H), 3.18 (m, 1H), 3.69 (m, 2H), 7.12 (d, 1H), 7.43 (d, 1H), 7.60 (d, 1H), 7.64 (t, 1H), 7.73 (t, 1H), 8.01 (bs, 3H), 8.57 (d, 1H). ¹³C NMR (DMSO-D6), δ (ppm): 7.2, 7.3, 13.0, 20.6, 42.6, 121.9, 122.8, 125.1, 126.8, 127.1, 127.2, 128.0, 128.8, 130.4, 133.5, 142.8, 155.8, 168.3.

### EXAMPLE 12

### Isopropylammonium salt of 2-(5-bromo-4-(1-cyclopropylnaphthalen-4-yl)-4H-1,2,4-triazol-3-ylthio)-acetic acid of formula 21

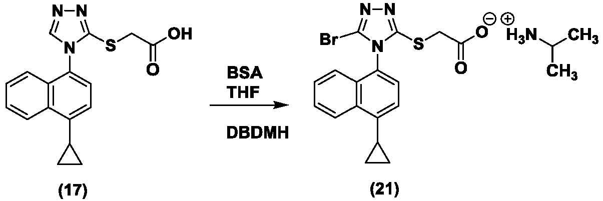

2-(4-(1-cyclopropylnaphthalen-4-yl)-4*H*-1,2,4-triazol-3-ylthio)acetic acid of formula 17 (10 g) was stirred up in tetrahydrofuran (50 ml) at 25°C, *N,O*-bis(trimethylsilyl)acetamide was added (7.5 g) and after 10 minutes 1,3-dibromo-5,5-dimethylhydantoin (6.3 g). The mixture was stirred at 55°C for 3 h, cooled to 25°C, sodium thiosulfate solution in water (20%, 20 ml), salt brine (10 ml) was added and the aqueous phase was separated after extraction. The organic phase was shaken again with a mixture of salt brine (10 ml) and water (10 ml). The organic phase was concetrated to half of its original volume, 2-methyltetrahydrofuran (50 ml) was added, the mixture was concentrated to half of its original volume and 2-methyltetrahydrofuran was added again and the mixture was concentrated to half of its original volume again. Acetone and isopropylamine (1.8 g) were added, 11.6 g (81.4%) of lesinurad isopropylammonium salt **(21)** was obtained after the crystallization.

### EXAMPLE 13

### Isopropylammonium salt of 2-(5-bromo-4-(1-cyclopropylnaphthalen-4-yl)-4H-1,2,4-triazol-3-ylthio)-acetic acid of formula 21

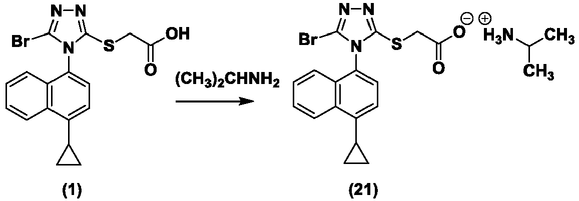

Lesinurad of formula **1** (0.2 g) was dissolved in 10 ml of an organic solvent, ispopropylamine (1.1 equivalents) was added and the mixture was stirred at 25°C. The separated product was filtered off, washed with the used solvent and dried by vacuum. The best results, judged in terms of yield and chemical purity, were achieved by the method of preparing lesinurad with isopropylamine of formula **21** by crystallization of ketones. Yields of crystalline salts were as follows: acetone (93.4%), 2-butanone (82.0%), 4-methyl-2-pentanone (84.6%), acetophenone (91.6%) and cyclohexanone (85.1%). However, the crystallization was also carried out from ethanol, ethyl acetate, 2-propanol, tetrahydrofuran, methanol, water and acetonitrile.

### EXAMPLE 14

### Guanidinium salt of 2-(5-bromo-4-(1-cyclopropylnaphthalen-4-yl)-4H-1,2,4-triazol-3-ylthio)-acetic acid of formula 22a

A 100 mg charge of lesinurad (0,2409 mmol) was dissolved in 7 ml of acetonitrile under intensive stirring at the room temperature. A solution of guanidine obtained by dissolution of 23 mg (0.2528 mmol) of guanidine carbonate in 0.3 ml of water was added to this solution. The obtained suspension was left to be stirred for 48 hours at the room temperature. The solid fraction was separated by filtration and washed with 5 ml of acetonitrile. The product was dried in a vacuum drier at the pressure of 130 mbar and the temperature of 40°C for 16 hours. X-ray powder pattern is shown in Fig. 2. The melting point according to DSC 157°C. Stoichiometric ratio of lesinurad:guanidine determined by means of ¹H NMR spectroscopy(1:1). HPLC purity 97.8%. Yield 86.35 mg (73.9%).

### EXAMPLE 15

### Guanidinium salt of 2-(5-bromo-4-(1-cyclopropylnaphthalen-4-yl)-4H-1,2,4-triazol-3-ylthio)-acetic acid of formula 22b

A 110 mg charge of lesinurad (0,2650 mmol) was dissolved in 8 ml of ethyl acetate under intensive stirring at the room temperature. A solution of guanidine obtained by dissolution of 25,1 mg (0.2692 mmol) of guanidine carbonate in 0.3 ml of water was added to this solution under intensive stirring. The obtained suspension was left to be stirred for 48 hours at the room temperature. The solid fraction was separat ned by filtration and washed with 5 ml of ethyl acetate. The product was dried in a vacuum drier at the pressure of 130 mbar and the temperature of 40°C for 16 hours. X-ray powder pattern is shown in Fig. 3. The melting point according to DSC 200°C. Stoichiometric ratio of lesinurad:guanidine determined by means of ¹H NMR spectroscopy(1:1). HPLC purity 97.6%. Yield 102.68 mg (79.9%).

### REFERENCE EXAMPLE 16

### Methyl 2-(5-bromo-4-(1-cyclopropylnaphthalen-4-yl)-4H-1,2,4-triazol-3-ylthio)acetate of formula 6a

Methanesulfonic acid (0.37 g, 3.85 mmol) was added to a suspension of methyl 2-(5-amino-4-(1-cyclopropylnaphthalen-4-yl)-4*H*-1,2,4-triazol-3-ylthio)-acetate (**5a**; 0.35 g, 1 mmol), KNO₂ (0.37 g, 4.3 mmol) and tetrabutylammonium tribromide (0.73 g, 1.5 mmol) in acetonitrile (5 ml) and the mixture was stirred at the laboratory temperature for 10 h. The mixture was diluted with dichloromethane, the obtained solution was washed with a saturated solution of NaHCO₃ and after washing with water it was dried with MgSO₄. The evaporation product containing 86% of the product was subsequently purified by means of column chromatography (silica gel, hexane-acetone 8 : 2). The amount of 0.31 g (74%) of a yellowish evaporation product was obtained with the HPLC purity of 97.9%, which was used for the synthesis of lesinurad **1** without further purification.

### REFERENCE EXAMPLE 17

### Ethyl 2-(5-bromo-4-(1-cyclopropylnaphthalen-4-yl)-4H-1,2,4-triazol-3-ylthio)acetate of formula 6b

Methanesulfonic acid (0.37 g, 5.8 mmol) was added to a suspension of ethyl 2-(5-amino-4-(1-cyclopropylnaphthalen-4-yl)-4*H*-1,2,4-triazol-3-ylthio)-acetate (**5b**; 0.37 g, 1 mmol), NaNO₂ (0.4 g, 5.8 mmol) and tetrabutylammonium tribromide (0.73 g, 1.5 mmol) in acetonitrile (5 ml) and the mixture was stirred at the laboratory temperature for 10 h. The mixture was diluted with ethyl acetate, the obtained solution was washed with a saturated solution of NaHCO₃ and after washing with water it was dried with MgSO₄. The evaporation product containing 89% of the product was subsequently purified by means of column chromatography (silica gel, hexane-acetone 8 : 2). The amount of 0.37 g (86%) of a colorless evaporation product was obtained with the HPLC purity of 99.2%, which was used for the synthesis of lesinurad **1** without further purification.

### REFERENCE EXAMPLE 18

### tert-Butyl 2-(5-bromo-4-(1-cyclopropylnaphthalen-4-yl)-4H-1,2,4-triazol-3-ylthio)acetate of formula 6c

Using the procedure described in Example 16, *tert*-butyl 2-(5-amino-4-(1-cyclopropylnaphthalen-4-yl)-4*H*-1,2,4-triazol-3-ylthio)acetate **5c** was used to obtain the bromo derivative **6c** in the 84% yield in the form of a colorless evaporation product with the HPLC purity of 99.1%, which was used for the synthesis of lesinurad **1** without further purification.

### REFERENCE EXAMPLE 19

### Ethyl 2-(5-bromo-4-(1-cyclopropylnaphthalen-4-yl)-4H-1,2,4-triazol-3-ylthio)acetate of formula 6c - screening of acids

The respective acid (5 mmol) was added to a suspension of ethyl 2-(5-amino-4-(1-cyclopropylnaphthalen-4-yl)-4*H*-1,2,4-triazol-3-ylthio)-acetate (**5b**; 0.37 g, 1 mmol), NaNO₂ (0.4 g, 5.8 mmol) and tetrabutylammonium tribromide (0.73 g, 1.5 mmol) in acetonitrile (5 ml) and the mixture was stirred at the laboratory temperature in the Radleys carousel device for 4 h. The mixture was diluted with dichloromethane, the obtained solution was washed with a saturated solution of NaHCO₃ and after washing with water it was analyzed with HPLC. The results of the screening of acids are presented in Table 5.

**Table 5**

| **Experiment** | **Acid** | **HPLC RS (%)** | | | | |
|---|---|---|---|---|---|---|
| | | **5b** | **6b** | **17** | **23** | **24** |
| A | acetic | 97.2 | - | - | 0.6 | - |
| B | trichloroacetic | 42.4 | 51.2 | 1.7 | - | 2.4 |
| C | dichloroacetic | 39.3 | 48.7 | 2.1 | - | 1.7 |
| D | monochloroacetic | 44.1 | 47.6 | 2.2 | - | 2.1 |
| E | bromoacetic | 9.7 | 87.5 | 0.6 | . | - |
| F | dibromoacetic | 12.1 | 74.4 | 0.7 | . | - |
| G | methane sulfonic | 9.7 | 89.9 | 0.2 | - | - |

### REFERENCE EXAMPLE 20

### Ethyl 2-(5-bromo-4-(1-cyclopropylnaphthalen-4-yl)-4H-1,2,4-triazol-3-ylthio)acetate of formula 6b - screening of solvents

Methanesulfonic acid (0.1 g, 1 mmol) was added to a suspension of ethyl 2-(5-amino-4-(1-cyclopropylnaphthalen-4-yl)-4*H*-1,2,4-triazol-3-ylthio)-acetate (**5b**; 0.37 g, 1 mmol), NaNO₂ (0.4 g, 5.8 mmol) and tetrabutylammonium tribromide (0.73 g, 1.5 mmol) in the respective solvent (5 ml) and the mixture was stirred at the laboratory temperature in the Radleys carousel device for 4 h. The mixture was diluted with dichloromethane, the obtained solution was washed with a saturated solution of NaHCO₃ and after washing with water it was analyzed with HPLC. The results of the screening of solvents are presented in Table 6.

**Table 6**

| **Experiment** | **Solvent** | **HPLC RS (%)** | | |
|---|---|---|---|---|
| | | **5b** | **6b** | **23** |
| A | MeCN | 48.2 | 37.7 | 2.6 |
| B | DMSO | 2.5 | 47.1 | 9.2 |
| C | THF | 57.3 | 5.8 | 37.7 |
| D | toluene | 67.2 | 20.6 | 12.1 |
| E | EtOAc | 83.1 | 7.2 | 2.1 |

### REFERENCE EXAMPLE 21

### Ethyl 2-(5-bromo-4-(1-cyclopropylnaphthalen-4-yl)-4H-1,2,4-triazol-3-ylthio)acetate of formula 6b

Tetrabutylammonium tribromide (7.3 g, 15 mmol) was added to a suspension of ethyl 2-(5-amino-4-(1-cyclopropylnaphthalen-4-yl)-4*H*-1,2,4-triazol-3-ylthio)-acetate (**5b**; 3.7 g, 10 mmol), KNO₂ (4.0 g, 47 mmol) and 18-crown-6 (0.25 g) in acetonitrile (50 ml) and the mixture was stirred for 10 min at the laboratory temperature. Then, methanesulfonic acid (0.3 ml) was added and the mixture was stirred at the laboratory temperature for 1 h. The mixture was diluted with ethyl acetate and the obtained solution was washed with salt brine and water and dried with MgSO₄. After evaporation, the amount of 3.3 g (76%) of a yellowish evaporation product was obtained, containing 93.2% of **6b** according to HPLC, which was used for the synthesis of lesinurad **1** without further purification.

### REFERENCE EXAMPLE 22

### Ethyl 2-(5-bromo-4-(1-cyclopropylnaphthalen-4-yl)-4H-1,2,4-triazol-3-ylthio)acetate of formula 6b - screening of tribromides

Methanesulfonic acid (0.1 g, 1 mmol) was added to a suspension of ethyl 2-(5-amino-4-(1-cyclopropylnaphthalen-4-yl)-4*H*-1,2,4-triazol-3-ylthio)-acetate (**5b**; 0.37 g, 1 mmol), NaNO₂ (0.4 g, 4.3 mmol), 15-crown-5 and the respective tribromide/pentabromide (1.5 mmol) in acetonitrile (5 ml) and the mixture was stirred at the laboratory temperature in the Radleys carousel device for 1 h. The mixture was diluted with dichloromethane, the obtained solution was washed with a saturated solution of NaHCO₃ and after washing with water it was analyzed with HPLC. The results of the screening of tribromides/pentabromides are presented in Table 7.

**Table 7**

| **Experiment** | **Tribromide/Pentabromide** | **HPLC RS (%)** | | |
|---|---|---|---|---|
| | | **5b** | **6b** | **23** |
| A | tetrabutylammonium tribromide | - | 94.7 | 4.2 |
| B | benzyltrimethylammonium tribromide | - | 96.4 | 3.7 |
| C | phenyltrimethylammonium tribromide | - | 97.2 | 2.2 |
| D | pyridinium tribromide | - | 98.1 | 1.4 |
| E | tetrabutylammonium pentabromide | - | 96.2 | 0.5 |
| F | benzyltrimethylammonium pentabromide | - | 96.1 | 0.4 |

### REFERENCE EXAMPLE 23

### Ethyl 2-(5-bromo-4-(1-cyclopropylnaphthalen-4-yl)-4H-1,2,4-triazol-3-ylthio)acetate of formula 6b - screening of a combination of tetraalkylammonium bromides with N-bromosuccinimide

To a suspension of ethyl 2-(5-amino-4-(1-cyclopropylnaphthalen-4-yl)-4*H*-1,2,4-triazol-3-ylthio)-acetate (**5b**; 0.37 g, 1 mmol), NaNO₂ (0.4 g, 4.3 mmol), and the respective tetraalkylammonium bromide (1.5 mmol) in acetonitrile (5 ml) *N*-bromosuccinimide (0.45 g, 2.5 mmol) was added and subsequently methanesulfonic acid (0.1 g, 1 mmol) and the mixture was stirred at the laboratory temperature in the Radleys carousel device for 1 h. The mixture was diluted with dichloromethane, the obtained solution was washed with a saturated solution of NaHCO₃ and after washing with water it was analyzed with HPLC. The results of the screening of tribromides/pentabromides are presented in Table 8.

**Table 8**

| **Experiment** | **Bromide** | **HPLC RS (%)** | | |
|---|---|---|---|---|
| | | **5b** | **6b** | **23** |
| A | tetrabutylammonium bromide | 2.2 | 90.1 | 6.2 |
| B | benzyltrimethylammonium bromide | 2.7 | 87.4 | 5.3 |
| C | benzyltriethylammonium bromide | 3.2 | 82.4 | 7.1 |
| D | phenyltrimethylammonium bromide | 4.8 | 77.7 | 5.9 |
| E | decyltrimethylammonium bromide | 4.1 | 87.4 | 4.4 |
| F | myristyltrimethylammonium bromide | 5.5 | 86.2 | 4.7 |
| G | didodecyl-dimethylammonium bromide | 5.1 | 83.3 | 7.7 |

### REFERENCE EXAMPLE 24

### Ethyl 2-(5-bromo-4-(1-cyclopropylnaphthalen-4-yl)-4H-1,2,4-triazol-3-ylthio)acetate of formula 6b

To a stirred suspension of benzyltriethylammonium bromide (0.4 g, 1.75 mmol) in acetonitrile (5 ml) bromine (0.25 g) was added dropwise and the mixture was stirred at the laboratory temperature for 2 hours. Then, ethyl 2-(5-amino-4-(1-cyclopropylnaphthalen-4-yl)-4*H*-1,2,4-triazol-3-ylthio)-acetate (**5b**; 0.37 g, 1 mmol), NaNO₂ (0.35 g, 5.0 mmol) and 18-crown-6 (0.05 g) was gradually added under stirring and the mixture was stirred for 10 min at the laboratory temperature. Then, methanesulfonic acid (0.05 ml) was added and the mixture was stirred at the laboratory temperature for 1 h. The mixture was diluted with ethyl acetate and the obtained solution was washed with salt brine and water and dried with MgSO₄. After evaporation, the amount of 0.45 g of a yellowish-brown evaporation product was obtained, containing in accordance with HPLC 88.2% of the compound of formula **6b,** which was subsequently purified by means of column chromatography (silica gel, hexane-acetone 8 : 2). The amount of 0.28 g (65%) of a yellowish evaporation product was obtained with the HPLC purity of 96.6%.

### REFERENCE EXAMPLE 25

### Ethyl 2-(5-bromo-4-(1-cyclopropylnaphthalen-4-yl)-4H-1,2,4-triazol-3-ylthio)acetate of formula 6b

Pyridinium tribromide (4.8 g, 15 mmol) was added to a suspension of ethyl 2-(5-amino-4-(1-cyclopropylnaphthalen-4-yl)-4*H*-1,2,4-triazol-3-ylthio)-acetate (**5b**; 3.7 g, 10 mmol), NaNO₂ (3.5 g, 50 mmol) and 18-crown-6 (0.25 g) in acetonitrile (50 ml) and the mixture was stirred for 10 min at the laboratory temperature. Then, methanesulfonic acid (0.3 ml) was added and the mixture was stirred at the laboratory temperature for 1 h. The mixture was diluted with ethyl acetate and the obtained solution was washed with salt brine and water and dried with MgSO₄. After evaporation, the amount of 3.7 g (85,6%) of a yellowish evaporation product was obtained, containing 96.1% of **6b** according to HPLC, which was used for the synthesis of lesinurad **1** without further purification.

### REFERENCE EXAMPLE 26

### Ethyl 2-(5-bromo-4-(1-cyclopropylnaphthalen-4-yl)-4H-1,2,4-triazol-3-ylthio)acetate of formula 6b

To a suspension of ethyl 2-(5-amino-4-(1-cyclopropylnaphthalen-4-yl)-4*H*-1,2,4-triazol-3-ylthio)-acetate (**5b**; 3.7 g, 10 mmol), NaNO₂ (3.5 g, 50 mmol) and PEG-400 (1 g) in acetonitrile (50 ml) pyridinium tribromide (4.8 g, 15 mmol) was added under cooling in an ice bath and the mixture was stirred for 10 min. Then, methanesulfonic acid (0.3 ml) was added and the mixture was stirred under cooling for 1 h. The mixture was diluted with ethyl acetate and the obtained solution was washed with salt brine and water and dried with MgSO₄. After evaporation, the amount of 3.5 g (81%) of a yellowish evaporation product was obtained, containing 95.1% of compound of formula **6b** according to HPLC, which was used for the synthesis of lesinurad **1** without further purification.

### EXAMPLE 27

### Isopropylammonium salt of 2-(5-bromo-4-(1-cyclopropylnaphthalen-4-yl)-4H-1,2,4-triazol-3-ylthio)acetic acid

To a suspension of ethyl 2-(5-bromo-4-(1-cyclopropylnaphthalen-4-yl)-4*H*-1,2,4-triazol-3-ylthio)-acetate (**6b**; 2.0 g, 4.4 mmol converted to 100% pure input compound), obtained according to Example 25, with the HPLC purity of 96.1% in ethanol (15 ml) a solution of NaOH (0.5 g) in water (5 ml) was added and the mixture was stirred at the laboratory temperature without any access of light for 8 h. Carborafin was added to the brown solution and after 2 hours of stirring the insoluble fraction was aspirated through kieselguhr. Ethanol was removed from the filtrate by distillation on a vacuum evaporator, the residue was supplemented with water to the volume of 10 ml and after acidifying with 5% HCl under cooling in an ice bath the mixture was stirred for 30 minutes. The insoluble fraction was aspirated, washed with water and dried. After dissolution in 4-methyl-2-pentanone (50 ml), isopropylamine (1 ml, 11.6 mmol) was added to the solution and the mixture was stirred without any access of light overnight. The separated product was aspirated, washed with 4-methyl-2-pentanone (2 x 5 ml) and dried by vacuum. The amount of 1.4 g (78.7% converted to 100% pure input compound) was obtained with the HPLC purity of 97.2%. Double crystallization from acetone provided 1.1 g of the salt (crystallization loss 22%) with the HPLC purity of 99.6%. ¹H NMR (DMSO-*d*₆), δ (ppm): 0.88 (m, 2H), 1.11 (d, 6H), 1.14 (m, 2H), 2.54 (m, 1H), 3.18 (m, 1H), 3.69 (m, 2H), 7.12 (d, 1H), 7.43 (d, 1H), 7.60 (d, 1H), 7.64 (t, 1H), 7.73 (t, 1H), 8.01 (bs, 3H), 8.57 (d, 1H). ¹³C NMR (DMSO-D6), δ (ppm): 7.2, 7.3, 13.0, 20.6, 42.6, 121.9, 122.8, 125.1, 126.8, 127.1, 127.2, 128.0, 128.8, 130.4, 133.5, 142.8, 155.8, 168.3.

### EXAMPLE 28

### Isopropylammonium salt of 2-(5-bromo-4-(1-cyclopropylnaphthalen-4-yl)-4H-1,2,4-triazol-3-ylthio)acetic acid

Using the procedure described in Example 27 with the use of LiOH, ethyl 2-(5-bromo-4-(1-cyclopropylnaphthalen-4-yl)-4*H*-1,2,4-triazol-3-ylthio)acetate **6b,** obtained according to Example 26, was converted in the 64% yield to the isopropylammonium salt with the purity of 99.8%.

### EXAMPLE 29

### 2-(5-bromo-4-(1-cyclopropylnaphthalen-4-yl)-4H-1,2,4-triazol-3-ylthio)acetic acid

A suspension of the isopropylammonium salt of 2-(5-bromo-4-(1-cyclopropylnaphthalen-4-yl)-4*H*-1,2,4-triazol-3-ylthio)acetic acid in tetrahydrofuran was acidified with 5% HCl to pH 2-3 under cooling and after dilution with water and cooling, the mixture was stirred at 5°C for 4 h without any access of light. The separated colorless crystals were aspirated and dried in vacuum of 10-12 Pa until a constant weight.

### EXAMPLE 30

### 5-amino-4-(1-cyclopropylnaphthalen-4-yl)-4H-1,2,4-triazol-3-ylthiol of formula 4

To a solution of 4-cyclopropyl-1-naphthylisothiocyanate of formula **3** (1.35 g, 6 mmol) in DMF (10 ml) S-methylthiosemicarbazide hydrogen iodide (1.5 g, 6.4 mmol) and to the obtained suspension a triethylamine (1.8 g, 18 mmol) was added under stirring in 15 minutes and the obtained mixture was stirred at the laboratory temperature for 4 hours. The mixture was poured into water (50 ml) and after acidifying with 5% HCl while stirring the mixture was stirred for additional 2 hours. The insoluble fraction was aspirated, washed with water and then dried in vacuum at the laboratory temperature. The amount of 1.6 g (94%) of a white solid was obtained of the m.t. 281-282°C (decomp.) with the HPLC purity of 98.4%. Recrystallisation of the sample from methyl acetate provided a sample of m.t. 283-284°C and the HPLC purity of 99.8%. ¹H NMR (500 MHz, DMSO-d₆): 12.89 (s, 1H, S*H*), 8.52 (d, 1H, *J* = 8.5 Hz, Ar*H*), 7.67 (ddd, 1H, *J* = 8.1, 6.8, 1.2 Hz, Ar*H*), 7.59 (ddd, 1H, *J* = 8.2, 6.9, 1.2 Hz, Ar*H*), 7.38 (s, 2H, Ar*H*), 7.31 (d, 1H, J = 8.2 Hz, Ar*H*), 5.88 (s, 2H, N*H₂*), 2.53 - 2.48 (m, 1H, cyclopropyl-C*H*), 1.17 - 1.11 (m, 2H, cyclopropyl-C*H₂*), 0.85 - 0.80 (m, 2H, cyclopropyl-C*H₂*). ¹³C NMR (500 MHz, DMSO-d₆): 164.87, 152.65, 141.26, 133.78, 129.67, 127.75, 127.55, 126.89, 126.50, 124.79, 122.95, 122.86, 12.92, 7.12, 6.87.

### REFERENCE EXAMPLE 31

### 5-amino-4-(1-cyclopropylnaphthalen-4-yl)-4H-1,2,4-triazole-3-thiol of formula 4

To a suspension of 4-cyclopropyl-1-naphthylisothiocyanate of formula **3** (0.23 g, 1 mmol) and S-methylthiosemicarbazide hydrogen iodide (0.25 g, 1.07 mmol) in 2 ml of a solvent (DMF, MeCN) a base (2 mmol) was added and the obtained mixture was stirred in vial at the laboratory temperature for 2 hours and the mixtures obtained were analysed by the mean of HPLC (HPLC RS). Than the content of each vial was poured on water (10 ml), acidified with 5% HCl and the precipitate was aspired, washed with water and dried. The crude products obtained were analysed by the mean of HPLC (HPLC SP). The results are presented in Table 9.

**Table 9**

| **Experiment** | **Solvent** | **Base** | **HPLC RS^{a}** | **HPLC RS** | **Yield** |
|---|---|---|---|---|---|
| A | DMF | - | 0 | 0 | 0 |
| B | DMF | Et₃N | 94.2% | 98.3% | 88% |
| C | DMF | EDIPA | 96.3% | 97.7% | 91% |
| D | DMF | K₂CO₃ | 93.1% | 99.0 | 87% |
| E | MeCN | Et₃N | 92.7% | 98.9% | 90% |
| F | MeCN | EDIPA | 95.8% | 99.1% | 92% |
| G | MeCN | K₂CO₃ | 96.7% | 99.7% | 95% |

| | | | | | |
|---|---|---|---|---|---|
| ^{a} - Solvent and base signals were not integrated. | | | | | |

### REFERENCE EXAMPLE 32

### 5-amino-4-(1-cyclopropylnaphthalen-4-yl)-4H-1,2,4-triazole-3-thiol of formula 4

To a suspension of 4-cyclopropyl-1-naphthylisothiocyanate of formula **3** (22.5 g, 100 mmol) and
S-methylthiosemicarbazide hydrogen iodide (25 g, 107 mmol) in MeCN (200 ml) triethyl amine (30 g, 296 mmol) was added by the linear dispenser in 1 hour and the mixture obtained was stirred at the laboratory temperature for 4 hours. The mixture was poured into water (500 ml) and acidified with 5% HCl while stirring and then the mixture was stirred for additional 12 hours. The insoluble fraction was aspirated, washed with water and then dried in vacuum at the laboratory temperature. The amount of 26.4 g (93.5%) of a white substance was obtained of the m.p. 284-284°C (decomp.) with the HPLC purity of 98.4%.

### REFERENCE EXAMPLE 33

### 5-amino-4-(1-cyclopropylnaphthalen-4-yl)-4H-1,2,4-triazole-3-thiol of formula 4

To a mixture of 4-cyclopropyl-1-naphthylisothiocyanate of formula **3** (2.25 g, 10 mmol) and S-dodecylthiosemicarbazide hydrogen bromide (4.0 g, 11.8 mmol) in MeCN (30 ml) triethyl amine (2.5 g, 25 mmol) was added while stirring and the mixture obtained was stirred at the laboratory conditions for 1 hour. The mixture was poured into water (200 ml) and was acidified with 5% HCl while stirring and then the mixture was stirred for additional 2 hours. The insoluble fraction obtained was aspirated, washed with water and then dried in vacuum at the laboratory temperature. The amount of 2.6 g (92%) of a white substance was obtained of the m.p. 281-282°C (decomp.) with the HPLC purity of 96.6%.

### REFERENCE EXAMPLE 34

### 5-amino-4-(1-cyclopropylnaphthalen-4-yl)-4H-1,2,4-triazole-3-thiol of formula 4

To a mixture of 4-cyclopropyl-1-naphthylisothiocyanate of formula **3** (2.25 g, 10 mmol) and S-dodecylthiosemicarbazide hydrogen bromide (4.0 g, 11.8 mmol) in acetonitrile (30 ml) triethylamine (2.5 g, 25 mmol) was added while stirring in 15 minutes and the mixture obtained was stirred at the laboratory temperature for 1 hour. The insoluble fraction was aspirated, the filtrate was diluted with water (100 ml) and acidified with 5% HCl while stirring and then the mixture was stirred for additional 2 hours. The insoluble fraction obtained was aspirated, washed with water and then dried in vacuum at the laboratory temperature. The amount of 2.2 g (78%) of a white substance was obtained of the m.p. 281-282°C (decomp.) with the HPLC purity of 97.7%.

### REFERENCE EXAMPLE 35

### 5-amino-4-(1-cyclopropylnaphthalen-4-yl)-4H-1,2,4-triazole-3-thiol of formula 4

To the mixture of 4-cyclopropyl-1-naphthylisothiocyanate of formula **3** (2.25 g, 10 mmol) and S-dodecylthiosemicarbazide hydrogen bromide (5.0 g, 11.8 mmol) in acetonitrile (30 ml) triethylamine (2.5 g, 25 mmol) was added while stirring in 15 minutes and the mixture obtained was stirred at the laboratory temperature for 1 hour. The insoluble fraction was aspirated, the filtrate was diluted with water (100 ml) and acidified with 5% HCl while stirring and then the mixture was stirred for additional 2 hours and the insoluble fraction obtained was aspirated, washed with water and then dried in vacuum at the laboratory temperature. The amount of 2.5 g (88.7%) of a white substance was obtained of the m.p. 28-282°C (decomp.) with the HPLC purity of 96.3%.

### REFERENCE EXAMPLE 36

### 5-amino-4-(1-cyclopropylnaphthalen-4-yl)-4H-1,2,4-triazole-3-thiol of formula 4

To the solution of 4-cyclopropyl-1-naphthylisothiocyanate of formula **3** (1.35 g, 6 mmol) in MeCN (10 ml) S-dodecylthiosemicarbazide hydrogen bromide (1.7 g, 6.5 mmol) was added and triethylamine (1.8 g, 18 mmol) was added to the suspension obtained while stirring in 15 minutes and the mixture obtained was stirred at the laboratory temperature for 4 hours. The mixture was poured into water (50 ml) and acidified with 5% HCl while stirring. The excluded greasy product was extracted with dichloromethane (5 x 10 ml) and dried with MgSO₄. The evaporation product was purified with chromatography on silica gel, as an eluent a dichloromethane was used first and then a mixture dichloromethane : methanol 99 : 1 to 90 : 10 (v/v). The amount of 0.9 g of the product **4** (53%) was obtained by the connection and evaporation of the corresponding fractions.

### REFERENCE EXAMPLE 37

### 5-amino-4-(1-cyclopropylnaphthalen-4-yl)-4H-1,2,4-triazole-3.thiol of formula 4

To the solution of 4-cyclopropyl-1-naphthylisothiocyanate of formula **3** (1.35 g, 6 mmol) in MeCN (10 ml) *S*-(4-*tert*-butyl benzyl)thiosemicarbazide hydrogen bromide (2.1 g, 6.6 mmol) was added and triethylamine (1.8 g, 18 mmol) was added to the suspension obtained while stirring in 15 minutes and the mixture obtained was stirred at the laboratory temperature for 4 hours. The mixture was poured into water (50 ml) and acidified with 5% HCl while stirring and the mixture was stirred at the laboratory temperature for 4 hours. The insoluble fraction obtained was aspirated, washed with water and then dried in vacuum at the laboratory temperature. The amount of 2.2 g (77.9%) of a white substance was obtained of the m.p. 280-282°C (decomp.) with the HPLC purity of 97.7%.

### REFERENCE EXAMPLE 38

### 5-amino-4-(1-cyclopropylnaphthalen-4-yl)-4H-1,2,4-triazole-3-thiol of formula 4

To the mixture of 4-cyclopropyl-1-naphthylisothiocyanate of formula **3** (2.25 g, 10 mmol) and *S*-(4-octyl benzyl)thiosemicarbazide hydrogen chloride (4.0 g, 12.1 mmol) in MeCN (50 ml) triethylamine (2.5 g, 25 mmol) was added and the mixture obtained was stirred at the laboratory temperature for 4 hours. The insoluble fraction was aspirated and washed with acetonitrile, the filtrate obtained was then poured into water (50 ml) and after acidification with 5% HCl the mixture was stirred for 4 hours. The insoluble fraction obtained was aspirated, washed with water and then dried in vacuum at the laboratory temperature. The amount of 2.55 g (90.4%) of a white substance was obtained of the m.p. 281-282°C (decomp.) with the HPLC purity of 94.4%.

### REFERENCE EXAMPLE 39

### Methyl 2-(5-amino-4-(1-cyclopropylnaphthalen-4-yl)-4H-1,2,4-triazol-3-ylthio)acetate of formula 5a

To the mixture of 4-cyclopropyl-1-naphthylisothiocyanate of formula **3** (4.5 g, 20 mmol) and S-dodecylthiosemicarbazide hydrogen bromide (8.0 g, 23.5 mmol) in acetonitrile (50 ml) triethylamine (5.0 g, 50 mmol) was added while stirring in 15 minutes and the obtained mixture was stirred at the laboratory temperature for 2 hours. The insoluble fraction was aspirated, more triethylamine (1 g, 10 mmol) was added to the filtrate and then a solution of methyl chloroacetate (3.25 g, 30 mmol) in acetonitrile (5 ml) was added by dripping while stirring and the mixture was stirred at the laboratory temperature overnight (12 hours). After adding the other fraction of methyl chloroacetate (0.5, 4.6 mmol) the mixture was stirred for additional 8 hours. The mixture was poured into water (250 ml) and extracted with methyl acetate. The extract was washed with water and dried with MgSO₄. After the evaporation, the evaporation product (6.5 g) was crystallised from methyl acetate and the amount of 5.2 g (73.4%) of white crystals was obtained of the m.p. 159-162°C and the HPLC purity of 97.6%. A little sample recrystallised from methyl acetate had the m.p. 132-163°C. ¹H NMR (500 MHz, CDCl₃): 8.54 (d, 1H, *J* = 8.4 Hz, Ar*H*), 7.67 (ddd, 1H, *J* = 8.2, 6.9, 1.2 Hz, Ar*H*), 7.59 (ddd, 1H, *J* = 8.2, 6.8, 1.1 Hz, Ar*H*), 7.44 (d, 1H, *J* = 8.4 Hz, Ar*H*), 7.42 (d, 1H, *J* = 7.5 Hz, Ar*H*), 7.37 (d, 1H, *J* = 7.6 Hz, Ar*H*), 4.30 (br.s., 2H, N*H₂*), 3.90 (d, 1H, J = 15.9 Hz, C*H₂*-S), 3.84 (d, 1H, *J* = 15.9 Hz, C*H₂*-S), 3.68 (s, 3H, O-C*H₃*), 2.45 - 2.40 (m, 1H, cyclopropyl-C*H*), 1.21 - 1.14 (m, 2H, cyclopropyl-C*H₂*), 0.90 - 0.83 (m, 2H, cyclopropyl-C*H₂*). ¹³C NMR (500 MHz, CDCl₃): 168.76, 155.72, 145.63, 143.08, 134.53, 129.27, 127.91, 127.22, 126.25, 125.34, 123.44, 122.30, 52.76, 34.54, 13.45, 6.90, 6.84.

### REFERENCE EXAMPLE 40

### Ethyl 2-(5-amino-4-(1-cyclopropylnaphthalen-4-yl)-4H-1,2,4-triazol-3-ylthio)acetate of formula 5b

To the mixture of 4-cyclopropyl-1-naphthylisothiocyanate of formula **3** (8.0 g, 35.6 mmol) and S-dodecylthiosemicarbazide hydrogen bromide (12.5 g, 36.8 mmol) in acetonitrile (75 ml) triethylamine (9.0 g, 90 mmol) was added while stirring in 15 minutes and the obtained mixture was stirred at the laboratory temperature for 2 hours. The insoluble fraction was aspirated and washed with acetonitrile (25 ml). More triethylamine (2.5 g, 25 mmol) was added to the filtrate and then a solution of ethyl bromoacetate (8 g, 48 mmol) was added in 30 minutes by dripping while stirring to the solution being cooled in the mixture of water-ice and the mixture was stirred at the laboratory temperature overnight (12 hours). After adding the another fraction of ethyl bromoacetate (2 g, 12 mmol) the mixture was stirred for additional 2 hours. The mixture was poured into water (250 ml), the excluded fraction was aspirated and washed with water (100 ml). The crude product obtained (13.9 g, the amount of 5b by the mean of HPLC 97.9%) was crystallised from ethyl acetate (130 ml) and the amount of 10.5 g (80%) of white crystals was obtained of the m.p. 129-132°C and the HPLC purity of 98.9%. ¹H NMR (500 MHz, CDCl₃): 8.54 (d, 1H, *J* = 8.4 Hz, Ar*H*), 7.66 (ddd, 1H, *J* = 8.2, 6.9, 1.3 Hz, Ar*H*), 7.59 (ddd, 1H, *J* = 8.2, 6.8, 1.2 Hz, Ar*H*), 7.44 (d, 1H, *J* = 8.5 Hz, Ar*H*), 7.42 (d, 1H, *J* = 7.5 Hz, Ar*H*), 7.37 (d, 1H, *J* = 7.6 Hz, Ar*H*), 4.28 (br.s., 2H, N*H₂*), 4.13 (q, 2H, *J* = 7.2 Hz, O-C*H₂*-CH₃), 3.88 (d, 1H, J = 15.8 Hz, C*H₂*-S), 3.82 (d, 1H, *J* = 15.9 Hz, C*H₂*-S), 2.45 - 2.40 (m, 1H, cyclopropyl-C*H*), 1.22 (t, 3H, *J* = 7.1 Hz, O-CH₂-C*H₃*), 1.19 - 1.16 (m, 2H, cyclopropyl-C*H₂*), 0.90 - 0.83 (m, 2H, cyclopropyl-C*H₂*). ¹³C NMR (500 MHz, CDCl₃): 168.26, 155.69, 145.67, 143.04, 134.52, 129.29, 127.89, 127.20, 126.28, 126.26, 125.32, 123.45, 122.31, 61.85, 34.79, 14.04, 13.44, 6.89, 6.84.

### REFERENCE EXAMPLE 41

### Ethyl 2-(5-amino-4-(1-cyclopropylnaphthalen-4-yl)-4H-1,2,4-triazol-3-ylthio)acetate of formula 5b

To the mixture of 4-cyclopropyl-1-naphthylisothiocyanate of formula **3** (2.25 g, 10 mmol) and S-methylthiosemicarbazide hydrogen iodide (2.5 g, 10.7 mmol) in DMF (30 ml) potash (5 g, 36 mmol) was added while stirring in 15 minutes and the obtained mixture was stirred at the laboratory temperature for 4 hours. Then a solution of ethyl bromoacetate (3.5 g, 21 mmol) in DMF (5 ml) was added by dripping while stirring to the reaction mixture and the mixture was stirred at the laboratory temperature overnight (12 hours). The mixture was poured into water (250 ml) and extracted with ethyl acetate. The extraction was washed with water and dried with MgSO₄. After the evaporation, the evaporation product obtained (2.5 g) was crystallised from ethyl acetate and the amount of 2.2 g (59.7%) of white crystals was obtained of the m.p. 130-132°C and the HPLC purity of 98.4%.

### REFERENCE EXAMPLE 42

### tert-Butyl 2-(5-amino-4-(1-cyclopropylnaphthalen-4-yl)-4H-1,2,4-triazol-3-ylthio)acetate of formula 5c

Using the procedure described in Example 41 with the use of *tert*-butyl bromoacetate instead of ethyl bromoacetate the composition of formula **5c** in the yield of 43% was obtained in the form of yellowish oil with the HPLC purity of 96.7%.

### ANALYTICAL METHODS

### High-performance liquid chromatography (HPLC)

HPLC chromatograms were measured using an EliteLachrom L-2450 device by Hitachi. A LichroCART 250-4 column was used, filled with an RP-18e stationary phase (5 µm) at 25°C. Acetonitrile (A) and a 0.02M solution of potassium dihydrogen phosphate whose pH was adjusted with phosphoric acid to 2.7 were used as a two-component mobile phase. The measurements were conducted in a gradient mode at the mobile phase flow rate of 1.0 ml/min. The composition of the phases at the beginning was 40% (A) and 60% (B), then the ratio of the constituents was maintained for 1 minute and after that changed to 80% (A) and 20% (B) in 14 minutes, then the ratio of the constituents was maintained for 15 minutes, after than it was changed to 40% (A) and 60% (B) in 3 minutes, after that the ratio of the constituents was maintained for 2 minutes (the total time of the analysis was 35 minutes). Detection at 290 nm was used. Acetonitrile was used as the solvent for the preparation of the analyzed samples, 10-20 µl of the prepared solution were injected.

### X-ray Powder Diffraction (XRPD)

The diffractograms were obtained using an X'PERT PRO MPD PANalytical powder diffractometer, used radiation CuKα (λ=1.542 Å), excitation voltage: 45 kV, anode current: 40 mA, measured range: 2 - 40° 2θ, increment: 0.01° 2θ at the dwell time at a reflection of 0.5 s, the measurement was carried out with a flat sample with the area/thickness of 10/0.5 mm. For the correction of the primary array 0.02 rad Soller slits, a 10 mm mask and a 1/4° fixed anti-dispersion slit were used. The irradiated area of the sample is 10 mm, programmable divergence slits were used. For the correction of the secondary array 0.02 rad Soller slits and a 5.0 mm anti-dispersion slit were used.

### NMR

The ¹H and ¹³C NMR spectra were measured using a Bruker Avance 500 spectrometer with the measuring frequency of 500.131 MHz, or. 125.762 MHz, respectively. The spectra were measured for solutions in DMSO-D6, ¹H chemical shifts were related to the internal standard of TMS δ = 0 ppm, ¹³C chemical shifts were related to the central signal DMSO-*d₅* (δ = 39.5 ppm).

### Differential Scanning Calorimetry (DSC)

The records of the solid crystalline forms of lesinurad were measured with the use of a DSC Pyris 1 device by Perkin Elmer. The sample charge in a standard Al pot was between 2.5-3 mg and the heating rate was 10°C/min. The temperature program that was used consists of 1 min of stabilization at the temperature of 0°C and then of heating up to 300°C at the heating rate of 10°C/min. As the carrier gas 4.0 N₂ was used at the flow of 20 ml/min.

## Claims

1. A method for preparing a compound of formula **1** or its salt 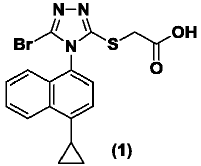 including
(a) a reaction of acid of formula **17** with a silylating agent in an organic solvent providing the intermediate of formula **18,** wherein R¹, R², R³ independently of one another stand for C₁-C₂₀ alkyl, C₁-C₂₀ alkenyl, C₃-C₁₀ cycloalkyl, C₃-C₁₀ cycloalkenyl or C₆-C₁₀ aryl; 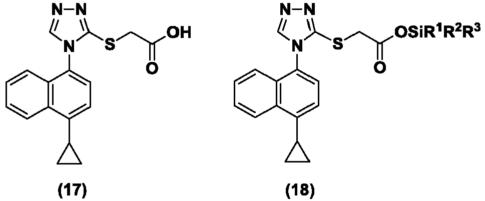
**(b)** bromination of the intermediate of formula **18** with the use of a brominating agent in an organic solvent, providing the intermediate of formula **19,** wherein R¹, R², R³ are as defined above; 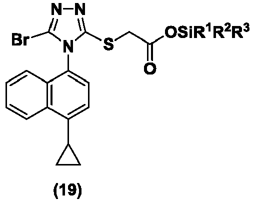
**(c)** deprotection of the trialkylsilyl group from the intermediate of formula **19,** providing the compound of formula **1;**
and
**(d)** isolation of the compound of formula **1** or its salt.

2. The method of preparing in accordance with claim 1, wherein the silylating agent is selected from the group: allyltrimethylsilane, *N*,*O*-bis(trimethylsilyl)acetamide, bis(trimethylsilyl)carbamate, *N,N*-bis(trimethylsilyl)formamide, *N,N-*bis(trimethylsilyl)methylamine, bis(trimethylsilyl) sulfate, *N,O-*bis(trimethylsilyl)trifluoroacetamide, *N,N'*-bis(trimethylsilyl)urea, (ethylthio)trimethylsilane, ethyltrimethylsilyl acetate, hexamethyldisilane, hexamethyldisilazane, hexamethyldisiloxane, hexamethyldisilthiane, (isopropenyloxy)trimethylsilane, methoxy-2-methyl-1-trimethylsiloxypropene, (methylthio)trimethylsilane, methyl-3-trimethylsiloxy-2-butenoate, *N*-methyl-*N-*trimethylsilyl acetamide, methyltrimethylsilyl acetate, *N*-methyl-*N*-trimethylsilyl heptafluorobutyramide, *N*-methyl-N-trimethylsilyl trifluoroacetamide, (phenyl-thio)trimethylsilane, 4-trimethylsiloxy-3-penten-2-one, *N*-(trimethylsilyl)acetamide, TMS-acetamide, trimethylsilyl acetate, trimethylsilyl azide, trimethylsilyl benzenesulfonate, trimethylsilyl cyanide, *N*-(trimethylsilyl)diethylamine, *N-*(trimethylsilyl)dimethylamine, trimethylsilyl-*N,N*-dimethylcarbamate, 1-(trimethylsilyl)imidazole, trimethylsilyl methanesulfonate, 4-(trimethylsilyl)morpholine, 3-trimethylsilyl-2-oxazolidinone, trimethylsilylperfluoro-1-butanesulfonate, TMS-nonaflate, trimethylsilyl trichloroacetate, trimethylsilyl trifluoroacetate, trimethylsilyl trifluoromethanesulfonate, 2-(trimethylsilyl)ethanol, 2-(trimethyl-silyl)ethoxymethylchloride.

3. The method of preparing in accordance with claim 2, wherein the silylating agent is selected from the group: *N,O*-bis(trimethylsilyl)acetamide, bis(trimethylsilyl)carbamate, *N,N*-bis(trimethylsilyl)formamide, *N,N-*bis(trimethylsilyl)methylamine, bis(trimethylsilyl) sulfate, *N,O-*bis(trimethylsilyl)trifluoracetamide and *N,N'*-bis(trimethylsilyl)urea.

4. The method of preparing in accordance with any of the preceding claims, wherein the brominating agent is selected from the group *N*-bromosuccinimide, pyridinium tribromide, benzyltrimethylammonium tribromide, 1,3-dibromo-5,5-dimethylhydantoine or dibromobarbituric acid.

5. The method of preparing in accordance with any of the preceding claims, wherein the solvent for preparing the intermediates of formulas **18** and **19** is selected from the group: acetonitrile, dimethyl formamide, dimethyl sulfoxide, acetone, 2-butanone, 4-methyl-2-pentanone, acetophenone, cyclohexanone, methanol, ethanol, isopropyl alcohol, ethylacetate, isopropyl acetate, tetrahydrofurane, 2-methyltetrahydrofurane, methylcyclopentyl ether, methyl-*t*-butyl ether, dichloromethane, cyclohexane, toluene.

6. The method of preparing in accordance with any of the preceding claims, wherein the intermediate of formula **18** is trimethylsilyl ester of formula **18a.**

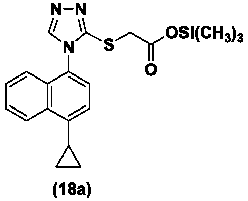

7. The method of preparing in accordance with any of the preceding claims, wherein 7 to 16 ml of tetrahydrofurane per gram of input substance of formula **17** and 1.1 to 2 equivalents of *N,O*-bis(trimethylsilyl)acetamide as the silylating agent are used.

8. The method of preparing in accordance with any of the preceding claims, wherein tetrahydrofurane as the solvent and 1.5 to 3 equivalents of *N*-bromosuccinimide as the brominating agent are used, preferably at the temperature of 40 to 60°C.

9. The method of preparing in accordance with any of the preceding claims, wherein the acid of formula **17** is prepared by the reaction of the compound of formula **11** with haloacetic acid, preferably with the bromoacetic or chloroacetic acid, in the presence of a base in the polar solvent.

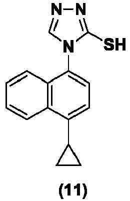

10. The method of preparing in accordance with any of the preceding claims, wherein the deprotection of the trialkylsilyl group from the intermediate of formula **19** is carried out by addition of water to a solution or extract of the compound of formula **19** in an organic solvent.

11. The method of preparing in accordance with any of the preceding claims, wherein the compound of formula **1** is isolated by crystallisation from the solution in organic solvents, which was obtained after deprotection of the trialkylsilyl group from the intermediate of formula **19,** as a free acid, or in the form of an ammonium salt after the addition of guanidine or isopropylamine.

12. The lesinurad salt of formula **21** or **22.**

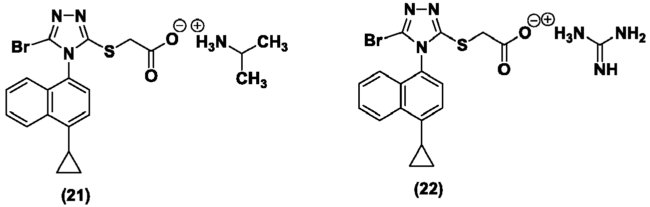

13. The crystalline isopropylammonium salt of lesinurad according to claim 12 of formula **21,** defined by the following characteristic reflections in the X-ray powder pattern measured with the use of CuKα radiation: 7.0; 16.8; 21.0; 22.9 and 24.1 ± 0.2° 2-theta.

14. The crystalline guanidinium salt of lesinurad according to claim 12 of formula **22,** defined by the following characteristic reflections in the X-ray powder pattern measured with the use of CuKα radiation: 7.1; 13.8; 18.5 and 24.5°± 0.2° 2-theta or 12.5; 14.7; 19.9; 23.0 and 26.4± 0.2° 2-theta.

15. The intermediate of formula **18, 18a** or **19,** wherein R¹, R² and R³ are as defined in the claim 1.

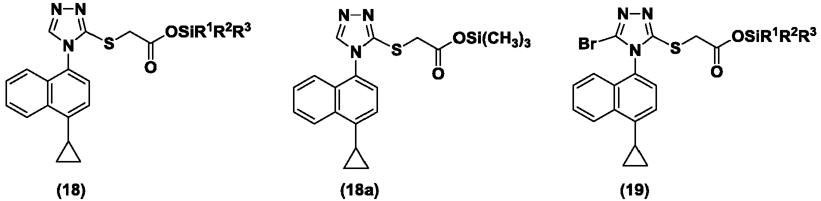

## Patentansprüche

1. Verfahren zur Herstellung einer Verbindung der Formel **1** oder ihres Salzes 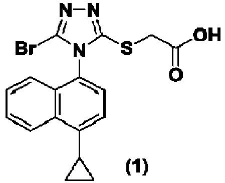 umfassend:
**(a)** Umsetzen der Säure der Formel **17** mit einem Silylierungsmittel in einem organischen Lösungsmittel, wobei das Zwischenprodukt der Formel **18** erhalten wird, worin R¹, R², R³ unabhängig voneinander für C₁-C₂₀-Alkyl, C₁-C₂₀-Alkenyl, C₃-C₁₀-Cycloalkyl, C₃-C₁₀-Cycloalkenyl oder C₆-C₁₀-Aryl stehen, 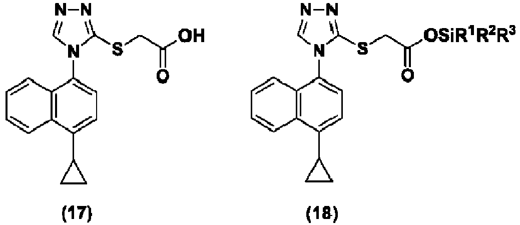
**(b)** Bromieren des Zwischenprodukts der Formel **18** unter Verwendung eines Bromierungsmittels in einem organischen Lösungsmittel, wobei das Zwischenprodukt der Formel **19** bereitgestellt wird, worin R¹, R², R³ wie oben definiert sind, 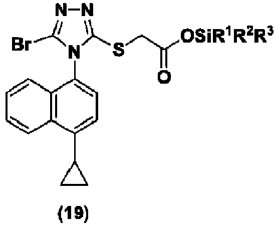
**(c)** Entschützen der Trialkylsilylgruppe des Zwischenprodukts der Formel **19,** wodurch die Verbindung der Formel **1** erhalten wird,
und
**(d)** Isolieren der Verbindung der Formel **1** oder ihres Salzes.

2. Herstellungsverfahren nach Anspruch 1, wobei das Silylierungsmittel aus der folgenden Gruppe ausgewählt ist: Allyltrimethylsilan, *N,O*-Bis(trimethylsilyl)acetamid, Bis(trimethylsilyl)carbamat, *N,N*-Bis(trimethylsilyl)formamid, *N,N*-Bis(trimethylsilyl) methylamin, Bis(trimethylsilyl)sulfat, *N,O*-Bis(trimethylsilyl)trifluoracetamid, *N,N'*-Bis(trimethylsilyl)harnstoff, (Ethylthio)trimethylsilan, ethyltrimethylsilylacetat, Hexamethyldisilan, Hexamethyldisilazan, Hexamethyldisiloxan, Hexamethyldisilthian, (Isopropenyloxy)trimethylsilan, Methoxy-2-methyl-1-trimethylsiloxypropen, (Methylthio)trimethylsilan, Methyl-3-trimethylsiloxy-2-butenoat, *N*-Methyl-*N*-trimethylsilylacetamid, Methyltrimethylsilylacetat, *N*-Methyl-*N*-trimethylsilylheptafluorbutyramid, *N*-Methyl-*N*-trimethylsilyltrifluoracetamid, (Phenylthio)trimethylsilan, 4-Trimethylsiloxy-3-penten-2-on, *N*-(Trimethylsilyl)acetamid, TMS-acetamid, Trimethylsilylacetat, Trimethylsilylazid, Trimethylsilylbenzolsulfonat, Trimethylsilylcyanid, *N*-(Trimethylsilyl)diethylamin, *N*-(Trimethylsilyl)dimethylamin, Trimethylsilyl-*N,N*-dimethylcarbamat, 1-(Trimethylsilyl)imidazol, Trimethylsilylmethansulfonat, 4-(Trimethylsilyl)morpholin, 3-Trimethylsilyl-2-oxazolidinon, Trimethylsilylperfluor-1-butansulfonat, TMS-Nonaflat, Trimethylsilyltrichloracetat, Trimethylsilyltrifluoracetat, Trimethylsilyltrifluormethansulfonat, 2-(Trimethylsilyl)ethanol, 2-(Trimethylsilyl)ethoxymethylchlorid.

3. Herstellungsverfahren nach Anspruch 2, wobei das Silylierungsmittel ausgewählt ist aus der Gruppe: *N,O*-Bis(trimethylsilyl)acetamid, Bis(trimethylsilyl)carbamat, *N,N-*Bis(trimethylsilyl)formamid, *N,N-*Bis(trimethylsilyl)methylamin, Bis(trimethylsilyl)sulfat, *N*,*O*-Bis(trimethylsilyl)trifluoracetamid und *N,N'-*Bis(trimethylsilyl)harnstoff.

4. Herstellungsverfahren nach einem der vorhergehenden Ansprüche, wobei das Bromierungsmittel ausgewählt ist aus der Gruppe *N*-Bromsuccinimid, Pyridiniumtribromid, Benzyltrimethylammoniumtribromid, 1,3-Dibrom-5,5-dimethylhydantoin oder Dibrombarbitursäure.

5. Herstellungsverfahren nach einem der vorhergehenden Ansprüche, wobei das Lösungsmittel zur Herstellung der Zwischenprodukte der Formeln **18** und **19** ausgewählt ist aus der Gruppe: Acetonitril, Dimethylformamid, Dimethylsulfoxid, Aceton, 2-Butanon, 4-Methyl-2-Pentanon, Acetophenon, Cyclohexanon, Methanol, Ethanol, Isopropylalkohol, Ethylacetat, Isopropylacetat, Tetrahydrofuran, 2-Methyltetrahydrofuran, Methylcyclopentylether, Methyl*-t*-butylether, Dichlormethan, Cyclohexan, Toluol.

6. Herstellungsverfahren nach einem der vorhergehenden Ansprüche, wobei das Zwischenprodukt der Formel **18** Trimethylsilylester der Formel **18a** ist.

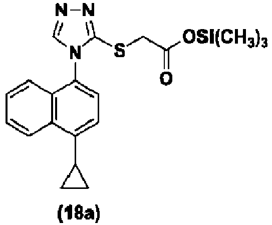

7. Herstellungsverfahren nach einem der vorhergehenden Ansprüche, wobei 7 bis 16 ml Tetrahydrofuran pro Gramm Einsatzstoff der Formel **17** und 1,1 bis 2 Äquivalente *N,O-*Bis(trimethylsilyl)acetamid als Silylierungsmittel verwendet werden.

8. Herstellungsverfahren nach einem der vorhergehenden Ansprüche, wobei Tetrahydrofuran als Lösungsmittel und 1,5 bis 3 Äquivalente *N*-Bromsuccinimid als Bromierungsmittel verwendet werden, vorzugsweise bei einer Temperatur von 40 bis 60 °C.

9. Herstellungsverfahren nach einem der vorhergehenden Ansprüche, wobei die Säure der Formel **17** hergestellt wird durch Umsetzung der Verbindung der Formel **11** mit Halogenessigsäure, vorzugsweise mit Bromessigsäure oder Chloressigsäure, in Gegenwart einer Base in dem polaren Lösungsmittel.

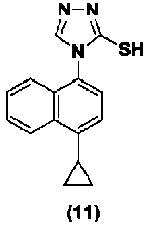

10. Herstellungsverfahren nach einem der vorhergehenden Ansprüche, wobei das Entschützen der Trialkylsilylgruppe des Zwischenprodukts der Formel **19** durch Zugabe von Wasser zu einer Lösung oder einem Extrakt der Verbindung der Formel **19** in einem organischen Lösungsmittel erfolgt.

11. Herstellungsverfahren nach einem der vorhergehenden Ansprüche, wobei die Verbindung der Formel **1** durch Kristallisation aus der Lösung in organischen Lösungsmitteln, welche nach Entschützen der Trialkylsilylgruppe des Zwischenprodukts der Formel **19** erhalten wurde, als freie Säure oder in Form eines Ammoniumsalzes nach Zugabe von Guanidin oder Isopropylamin isoliert wird.

12. Lesinurad-Salz der Formel **21** oder **22.**

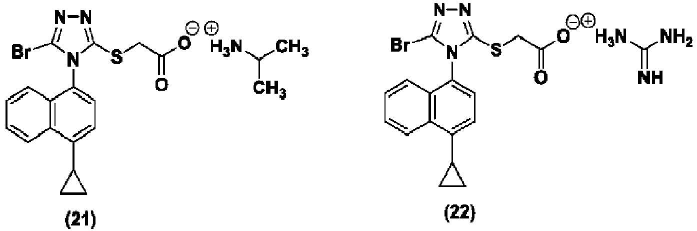

13. Kristallines Isopropylammoniumsalz von Lesinurad der Formel **21** nach Anspruch 12, definiert durch die folgenden charakteristischen Reflexionen im Röntgenpulverdiagramm, gemessen unter Verwendung von CuKa-Strahlung: 7,0; 16,8; 21,0; 22,9 und 24,1 ± 0,2° 2-Theta.

14. Kristallines Guanidiniumsalz von Lesinurad der Formel **22** nach Anspruch 12, definiert durch die folgenden charakteristischen Reflexionen im Röntgenpulverdiagramm, gemessen unter Verwendung von CuKα-Strahlung: 7.1; 13,8; 18,5 und 24,5°± 0,2° 2-Theta oder 12,5; 14,7; 19,9; 23,0 und 26,4± 0,2° 2-Theta.

15. Zwischenprodukt der Formel **18, 18a** oder **19,** worin R¹, R² und R³ wie in Anspruch 1 definiert sind.

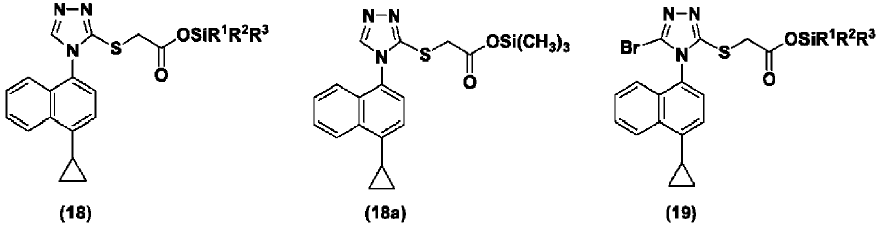

## Revendications

1. Un procédé de préparation d'un composé de formule **1** ou de son sel, 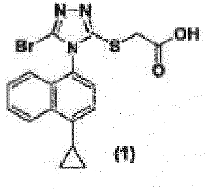 comprenant
(a) une réaction d'un acide de formule **17** avec un agent de silylation dans un solvant organique fournissant l'intermédiaire de formule **18,** dans laquelle : R¹, R², R³, indépendamment les uns des autres, représentent un groupe de la liste suivante: alkyle en C₁-C₂₀, alcényle en C₁-C₂₀, cycloalkyle en C₃-C₁₀, cycloalcényle en C₃-C₁₀ ou aryle en C₆-C₁₀; 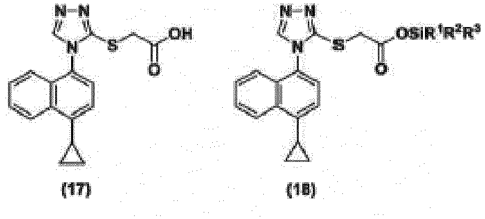
(b) bromation de l'intermédiaire de formule **18** avec utilisation d'un agent de bromation dans un solvant organique, fournissant l'intermédiaire de formule **19,** formules dans lesquelles:
R¹, R², R³ sont tels que définis ci-dessus; 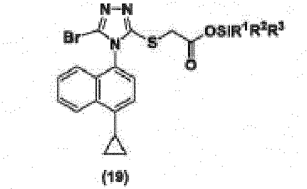
(c) la déprotection du groupe trialkylsilyle de l'intermédiaire de formule **19,** fournissant le composé de formule **1;**
et
(d) l'isolement du composé de formule **1** ou de son sel.

2. Le procédé de préparation selon la revendication 1, dans lequel l'agent de silylation est choisi dans le groupe comprenant: allyltriméthylsilane, N,O-bis-triméthylsilyl)acétamide, le bis-(triméthylsilyl)carbamate, le N,N-bis-(triméthylsilyl)formamide, le N,N-bis-(triméthylsilyl)méthylamine, le bis(triméthylsilyl)sulfate, le N-O-bis(triméthylsilyl)trifluoroacétamide, le N-N'-bis(triméthylsilyl)urée, (ethylthio)triméthylsilane, l'acétate d'éthyltriméthylsilyl, l'héxaméthyldisilane, l'héxaméthyldisilazane, l'héxaméthyldisiloxane, l'héxaméthyldisilthiane, (isopropényloxy)triméthylsilane, le méthoxy-2-méthyl-1-triméthylsiloxypropène, le (méthylthio)triméthylsilane, le méthyl-3-triméthylsiloxy-2-buténoate, le N-méthyl-N-triméthylsilyl acétamide, l'acétate de méthyltriméthylsilyl, N-méthyl-N-triméhylsilyl heptafluorobutyramide, le N-méthyl-N-triméthylsilyl trifluoroacétamide, le (phénylthio)triméthylsilane, le 4-triméthylsiloxy-3-pentène-2-one, le N-(triméthylsilyl)acétamide, le TMS-acétamide, l'acétate de triméthylsilyle, le triméthylsilylazide, le benzenesulfonate de triméthylsilyle, le cyanure de triméthylsilyle, la N-(triméthylsilyl)diéthylamine, la N-(triméthylsilyl)diméthylamine, le triméthylsilyl-N,N-diméthylcarbamate, le 1-(triméthylsilyl)imidazole, le méthanesulfonate de triméthylsilyle, le 4-(triméthylsilyl)morpholine, le 3-triméthylsilyl-2-oxazolidinone, le sulfonate de triméthylsilyl-perfluoro-1-butane, le TMS-nonaflate, le trichloroacétate de triméthylsilyle, le trifluoroacétate triméthylsilyle, le sulfonate de triméthylsilyl-trifluorométhane, le 2-(triméthylsilyl)-éthanol, le chlorure de (2-triméthylsylyl)-éthoxyméthyle.

3. Le procédé de préparation selon la revendication 2, dans lequel l'agent de silylation est choisi dans le groupe comprenant le N,O-bis-(triméthylsilyl)-acétamide, le bis-(triméthylsilyl)carbamate, le N,N-bis-(triméthylsilyl)formamide, le N,N-bis-triméthylsilyl)méthylamine, le bis-(triméthylsilyl)sulfate, le N,O-bis-(triméthylsilyl)trifluoracétamide et le N,N'-bis-(triméthylsilyl)urée.

4. Le procédé de préparation selon l'une quelconque des revendications précédentes, dans lequel l'agent de bromation est choisi dans le groupe comprenant le N-bromosuccinimide, le tribromure de pyridinium, le tribromure de benzyltriméthyl-ammonium, l'acide 1,3-dibromo-5,5-diméthylhydantoïne ou l'acide dibromo-barbiturique

5. Le procédé de préparation selon l'une quelconque des revendications précédentes, dans lequel le solvant pour préparer les intermédiaires de formules **18** et **19** est choisi dans le groupe comprenant: l'acétonitrile, le diméthylformamide, le diméthylsulfoxyde, l'acétone, le 2-butanone, le 4-méthyl-2-pentanone, acétophénone, le cyclohexanone, le méthanol, l'éthanol, l'alcool isopropylique, l'acétate d'éthyle, l'acétate d'isopropyle, le tétrahydrofurane, le 2-méthyltétrahydrofurane, le méthylcyclopentyléther, le méthyl-t-butyléther, le dichlorométhane, le cyclohexane, le toluène.

6. Le procédé de préparation selon l'une quelconque des revendications précédentes, dans lequel l'intermédiaire de formule **18** est un ester triméthylsilylique de formule **18a.**

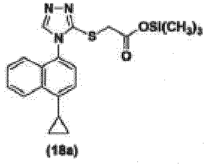

7. Le procédé de préparation selon l'une quelconque des revendications précédentes, dans lequel sont utilisés 7 à 16 ml de tétrahydrofurane par gramme de substance de formule **17** introduite et 1,1 à 2 équivalents de N,O-bis-(triméthylsilyl)acétamide en tant qu'agent de silylation.

8. Le procédé de préparation selon l'une quelconque des revendications précédentes, dans lequel on utilise du tétrahydrofurane comme solvant et 1,5 à 3 équivalents de N-bromosuccinimide comme agent de bromation, de préférence à une température de 40 à 60°C.

9. Le procédé de préparation selon l'une quelconque des revendications précédentes, dans lequel l'acide de formule **17** est préparé par réaction du composé de formule **11** avec de l'acide haloacétique, de préférence avec l'acide bromoacétique ou l'acide chloroacétique, en présence d'une base dans le solvant polaire.

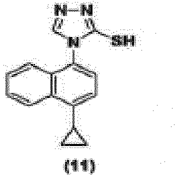

10. Le procédé de préparation selon l'une quelconque des revendications précédentes, dans lequel la déprotection du groupe trialkylsilyle de l'intermédiaire de formule **19** est effectuée par addition d'eau à une solution ou à un extrait du composé de formule **19** dans un solvant organique.

11. Le procédé de préparation selon l'une quelconque des revendications précédentes, dans lequel le composé de formule **1** est isolé par cristallisation de la solution dans des solvants organiques, qui est obtenue après déprotection du groupe trialkylsilyle de l'intermédiaire de formule **19,** sous forme d'acide libre ou sous la forme d'un sel d'ammonium après addition de guanidine ou d'isopropylamine.

12. Le sel de lesinurad de formule **21** ou **22.**

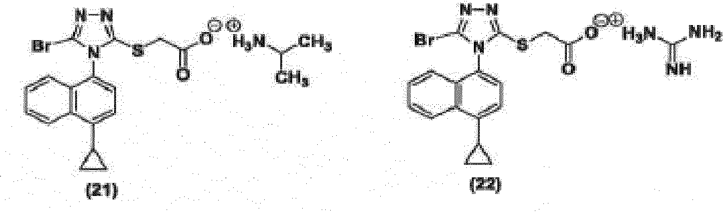

13. Le sel d'isopropylammonium cristallin de lésinurad selon la revendication 12 de formule **21,** défini par les réflexions caractéristiques suivantes du diagramme de poudre aux rayons X mesuré avec utilisation du rayonnement CuKα: 7,0; 16,8; 21,0; 22,9 et 24,1 ± 0,2 ° 2-thêta.

14. Le sel de guanidinium cristallin de lesinurad de formule **22,** selon la revendication 12, défini par les réflexions caractéristiques suivantes du diagramme de poudre aux rayons X mesuré avec utilisation du rayonnement CuKα: 7,1; 13,8; 18,5 ; et 24,5 ° ± 0,2 ° 2-thêta ou 12,5; 14,7; 19,9; 23,0 et 26,4 ± 0,2 ° 2-thêta.

15. Les intermédiaires de formules **18, 18a** ou **19,** dans lesquelles R¹, R² et R³ sont tels que définis dans la revendication 1.
